# NEUE EUROPÄISCHE PATENTSCHRIFT

(11) **EP 0 743 367 B2**
(45) Veröffentlichungstag und Bekanntmachung des Hinweises auf die Entscheidung über den Einspruch: **26.10.2005**
(45) Hinweis auf die Patenterteilung: 07.08.2002
(21) Anmeldenummer: 96107942.3
(22) Anmeldetag: 17.05.1996
(51) Int. Cl.: C12Q 1/68

(54) **Verfahren zur Genexpressionsanalyse**
Gene expression analysis
Méthode pour l'analyse de l'expression génétique

(30) Priorität: 19.05.1995 DE 19518505
(43) Veröffentlichungstag der Anmeldung: 20.11.1996
(60) Teilanmeldung: 01129103.6
(73) Patentinhaber: AXARON Bioscience AG, 69120 Heidelberg (DE)
(72) Erfinder: Fischer, Achim, Dr., 69120 Heidelberg (DE)
(74) Vertreter: Lahrtz, Fritz

(56) Entgegenhaltungen:
- WO-A-93/18176
- KOTSINAS A. ET AL: "Relative expression of wild-type and activated Ki-ras2 oncogene in colorectal carcinomas." INTERNATIONAL JOURNAL OF ONCOLOGY, (1993) 3/5 (841-845). , XP002141822
- WANG A ET AL: "A GENE EXPRESSION SCREEN" PROCEEDINGS OF THE NATIONAL ACADEMY OF SCIENCES OF USA,US,NATIONAL ACADEMY OF SCIENCE. WASHINGTON, Bd. 88, 1. Dezember 1991 (1991-12-01), Seiten 11505-11509, XP000619920 ISSN: 0027-8424
- STONE B AND WHARTON W: "TARGETED RNA FINGERPRINTING: THE CLONING OF DIFFERENTIALLY-EXPRESSED cDNA FRAGMENTS ENRICHED FOR MEMBERS OF THE ZINC FINGER GENE FAMILY" NUCLEIC ACIDS RESEARCH,GB,OXFORD UNIVERSITY PRESS, SURREY, Bd. 13, Nr. 22, 1. Januar 1994 (1994-01-01), Seiten 2612-2618, XP002076945 ISSN: 0305-1048
- FROHMAN M A ET AL: "RAPID PRODUCTION OF FULL-LENGTH CDNAS FROM RARE TRANSCRIPTS: AMPLIFICATION USING A SINGLE GENE-SPECIFIC OLIGONUCLEOTIDE PRIMER" PROCEEDINGS OF THE NATIONAL ACADEMY OF SCIENCES OF USA,US,NATIONAL ACADEMY OF SCIENCE. WASHINGTON, Bd. 85, 1. Dezember 1988 (1988-12-01), Seiten 8998-9002, XP000604678 ISSN: 0027-8424
- BAUER ET AL: "IDENTIFICATION OF DIFFERENTIALLY EXPRESSED mRNA SPECIES BY AN IMPROVED DISPLAY TECHNIQUE" NUCLEIC ACIDS RESEARCH,GB,OXFORD UNIVERSITY PRESS, SURREY, Bd. 21, Nr. 18, 11. September 1993 (1993-09-11), Seiten 4272-4280, XP002086977 ISSN: 0305-1048
- FISCHER A ET AL: "RESTRICTION FRAGMENT LENGTH POLYMORPHISM-COUPLED DOMAINDIRECTED DIFFERENTIAL DISPLAY: A HIGHLY EFFICIENT TECHNIQUE FOR EXPRESSION ANALYSIS OF MULTIGENE FAMILIES" PROCEEDINGS OF THE NATIONAL ACADEMY OF SCIENCES OF USA,US,NATIONAL ACADEMY OF SCIENCE. WASHINGTON, Bd. 92, 1. Juni 1995 (1995-06-01), Seiten 5331-5335, XP002059940 ISSN: 0027-8424

(1) ALLGEMEINE ANGABEN:
   (i) ANMELDER:
      (A) NAME: Max-Planck-Gesellschaft zur Foerderung der Wissenschaften e.v. Berlin
      (B) STRASSE: Hofgartenstr. 2
      (C) ORT: Muenchen
      (E) LAND: DE
      (F) POSTLEITZAHL: 80539
   (ii) BEZEICHNUNG DER ERFINDUNG: Verfahren zur Genexpressionsanalyse
   (iii) ANZAHL DER SEQUENZEN: 14
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANKELDENUMMER: DE 19518505.6
      (B) ANMELDETAG: 19-MAY-1995
(2) ANGABEN ZU SEQ ID NO: 1:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 1:
(2) ANGABEN ZU SEQ ID NO: 2:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 2:
   ACCTACGTGC AGATTTTTTT TTTTTTTTV   29
(2) ANGABEN ZU SEQ ID NO: 3:
   (i) SEQUENZKENNZEICHEN:
      (A) LÄNGE: 23 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
   CACGATCTGA GGCCGATCGA TTC 23
(2) ANGABEN ZU SEQ ID NO: 4:
   (i) SEQUENZKENNZEICHEN:
      (A) LÄNGE: 20 Basenpaare
      (B) ART: Nucleotid
      (C) STRANGFORN: Einzelstrang
      (D) TOPOLOGIE: linear
   (xi) SEQUENZBESCHREIBUNG: SEQ ID NO: 4:
   TCACATGCTA AGTCTCGCGA 20
(2) ANGABEN ZU SEQ ID NO: 5:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 5:
(2) ANGABEN ZU SEQ ID NO: 6:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 6:
   GATCAAGMGS ATCGAGAA 18
(2) ANGABEN ZU SEQ ID NO: 7:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 7:
   GATGAAGMGS ATCGAGAA 18
(2) ANGABEN ZU SEQ ID NO: 8:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 8:
   GATCAAGCAY ATCGAGAA 18
(2) ANGABEN ZU SEQ ID NO: 9:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 9:
(2) ANGABEN ZU SEQ ID NO: 10:
   (i) SEQUENZIENNZEICHEN:
      (A) LÄNGE: 32 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 10:
   ACTCGATTCT CAACCCGAAA GTATAGATCC CA 32
(2) ANGABEN ZU SEQ ID NO: 11:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 11:
   TGGGATCTAT ACTTTCAA 18
(2) ANGABEN ZU SEQ ID NO: 12:
   (i) SEQUENZKENNZEICHEN:
      (A) LÄNGE: 15 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 12:
   MGSCAGGTSA CSTWC 15
(2) ANGABEN ZU SEQ ID NO: 13:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 13:
(2) ANGABEN ZU SEQ ID NO: 14:
   (i) SEQUENZKENNZEICHEN:
      (A) LÄNGE: 60 Aminosäuren
      (B) ART: Aminosäure
      (C) STRANGFORM:
      (D) TOPOLOGIE: linear
   (xi) SEQUENZBESCHREIBUNG: SEQ ID NO: 14:

## Beschreibung

Die vorliegende Erfindung betrifft ein Verfahren zur allgemeinen Genexpressionsanalyse sowie ein Verfahren zur differenziellen Analyse der Expression von Mitgliedern einer Genfamilie.

Die molekulare Grundlage von zahlreichen biologischen Prozessen ist die koordinierte Aktivierung bzw. Inaktivierung bestimmter Gene oder Gruppen von Genen in einer Zelle, einem Organ oder einem Organismus. Dabei ist die Zelle, das Organ bzw. der Organismus in einem bestimmten physiologischen Zustand, einem Differenzierungsgrad und bei bestimmten äußeren Einflüssen durch einen spezifischen Expressionsstatus gekennzeichnet. Die Kenntnis dieses Expressionsstatus, d. h. des Aktivierungsgrads aller Gene oder bestimmter Gruppen von Genen, ist von zentraler Bedeutung für die Beantwortung vieler biologischer Fragen. Das Ziel einer Methode, mittels derer Aussagen über den Expressionsstatus gewonnen werden sollen, ist es somit daher einerseits, die Aktivität aller Gene als auch die Aktivität einer bestimmten Teilmenge von Genen festzustellen. Als Teilmengen kommen insbesondere Genfamilien in Frage. Genfamilien sind Gruppen von Genen, die sich häufig funktionell durch einen bestimmten Aufgabentyp auszeichnen, den die Genprodukte in einer Zelle übernehmen, und denen strukturell ein oder mehrere konservierte Bereiche (Domänen) gemeinsam zu eigen sind. Beispiele von Genfamilien sind die MADS-Box- und die Homeobox-Genfamilie sowie weitere Transkriptionsfaktor-Familien. Von medizinischem Interesse sind beispielsweise die Cyclin-, Cytokin- und Globin-Genfamilien.

Aus dem Stand der Technik ist die Analyse des Expressionsstatus von Genen bekannt. Die Differential Display RT-PCR (DDRT) ist beispielsweise ein Verfahren zur Analyse differenzieller Genexpression, wobei mRNA aus einem Gewebe durch Verwendung eines cDNA-Primers mit 3'-Extension (vorzugsweise zwei Basen) durch Reverse Transkription (RT) in (vorzugsweise 12) Subpopulationen von cDNA umgeschrieben wird. Aus dieser cDNA werden durch Verwendung kurzer, vorzugsweise zehn Nucleotide langer Primer mit Zufallssequenz PCR-Produkte mit transkriptspezifischer Länge erzeugt, die auf Sequenzgelen aufgetrennt werden können. Ist die Zahl der verwendeten Primer-Kombinationen groß genug, können statistisch so gut wie alle im Gewebe vorhandenen Transkripte nachgewiesen werden. Werden die aus zwei oder mehr Geweben gewonnenen PCR-Produkte nebeneinander auf ein Gel aufgetragen, können auf diese Weise Expressionsunterschiede unmittelbar sichtbar gemacht werden. Differenziell erscheinende Banden können zur weiteren Analyse aus dem Gel ausgeschnitten, reamplifiziert und kloniert werden.

Weiterhin kann zur Anreicherung der Amplifikationsprodukte für eine bestimmte Untergruppe aller mRNA-Moleküle, z. B. Mitglieder einer bestimmten Genfamilie, ein Primer, der eine Genfamilien-spezifische Sequenz aufweist, mit einem Zufallsprimer kombiniert werden. Diese Technik der DDRT ist beispielsweise beschrieben bei Liang und Pardee (1992) Science 257, 967-971; Liang et al (1993), Nucleic Acids Res. 21, 3269-3275; Bauer et al (1993) Nucleic Acids Res. 21, 4272-4280; Stone und Wharton (1994), Nucleic Acids Res. 22, 2612-2618 und Wang und Feuerstein (1995) Biotechniques 18,448-452 sowie WO 93/18176 und DE 43 17 414.

Das experimentelle Design der DDRT führt jedoch zu einer Reihe von Nachteilen. So sind die Bandenmuster oft nur schlecht reproduzierbar. Auch der Einsatz längerer Zufallsprimer von z. B. 20 Basen Länge konnte das Problem der schlechten Reproduzierbarkeit nicht zufriedenstellend lösen (Ito et al., (1994), FEBS Lett. 351, 231-236). Weiterhin ist durch die hohe Zahl der notwendigen Primerkombinationen sowie durch die Notwendigkeit, die Reproduzierbarkeit durch Replikaansätze zu erhöhen, ein extremer Aufwand erforderlich. Zudem erhält man oft einen hohen Anteil von falsch-positiven Ergebnissen. Darüber hinaus konnte gezeigt werden, daß seltene Transkripte in vielen DDRT-Experimenten nicht detektiert werden können (Bertioli et al. (1995), Nucleic Acids Res. 23, 4520-4523).

Auch die im Stand der Technik beschriebene Sequenzierung ausgewählter Banden ist problematisch, da DDRT-Produkte oft an beiden Enden vom gleichen Primer flankiert werden, so daß eine Direkt-Sequenzierung nicht möglich ist und ein zusätzlicher Klonierungsschritt erforderlich wird. Aufgrund der Verwendung kurzer Primer ist selbst, wenn das Produkt von zwei verschiedenen Primern flankiert wird, ein weiterer Reamplifikationsschritt mit gegenüber den DDRT-Primern 5'-seitig verlängerten Primermolekülen erforderlich. Schließlich kann aufgrund der Verwendung von Zufallsprimern auch nie mit Sicherheit davon ausgegangen werden, daß alle Transkripte einer Zelle von den eingesetzten Primerkombinationen erkannt werden können. Dies gilt - selbst bei Anwendung einer hohen Anzahl von Primern - sowohl für einen Ansatz, der die Gesamtheit aller Transkripte erfassen soll, als auch für einen Ansatz, der auf die Analyse einer Subpopulation der Transkripte, etwa eine Genfamilie, ausgerichtet ist.

Song und Osborn beschreiben in Plant Molecular Biology 26 (1994), 1065-1071, ein Verfahren zur Untersuchung der Expression homologer Gene in Pflanzenpolyploiden, bei dem die Technik der RT-PCR und der RFLP (Restriktionsfragment-Längenpolymorphismus)-Analyse mit einander kombiniert werden. Dabei wird aus RNA durch Reverse Transkription eine cDNA hergestellt, die unter Verwendung von zwei genspezifischen Primern amplifiziert wird. Die Amplifikationsprodukte werden durch Endonucleasespaltung transkriptspezifisch verkürzt, ihrer Länge nach aufgetrennt und durch anschließende Klonierung und Sequenzierung analysiert. Auch dieses Verfahren hat den Nachteil einer geringen Sensitivität, da zur Identifizierung der Expressionsprodukte ein zusätzlicher Klonierungsschritt erforderlich ist. Ein weiterer Nachteil dieses Verfahrens besteht darin, daß genspezifische Sequenzinformationen über mindestens zwei Regionen innerhalb der analysierten Gene vorliegen müssen, um geeignete Primer entwerfen zu können.

Die der vorliegenden Erfindung zugrundeliegende Aufgabe bestand somit darin, ein Verfahren zur Genexpressionsanalyse bereitzustellen, bei dem die Nachteile des Standes der Technik mindestens teilweise vermieden werden können, insbesondere ein Verfahren, bei dem reproduzierbare Ergebnisse erhalten werden, das eine ausreichende Sensitivität aufweist, um auch seltene Transkripte zu detektieren, und das eine Identifizierung von Amplifikationsprodukten ohne zusätzlichen Klonierungsschritt ermöglicht.

Zur Lösung dieser Aufgabe wird im Rahmen der vorliegenden Erfindung ein Verfahren zur Genexpressionsanalyse bereitgestellt, gekennzeichnet durch die Schritte:
(a) Isolieren von mRNA-Molekülen aus einer oder mehreren zu analysierenden Gewebeproben,
(b) Synthetisieren von cDNA-Erststrangmolekülen aus den mRNA-Molekülen,
(c) Synthetisieren von cDNA-Zweitstrangmolekülen,
(d) Spalten der cDNA-Moleküle aus (c) mit mindestens einer Restriktionsendonuklease,
   wobei die Restriktionsendonuklease eine vierbasige Erkennungssequenz aufweist,
(e) Anfügen von Linkem an die durch den Restriktionsverdau gewonnene cDNA-Fragmente,
(f) Selektive Amplifikation eines Teils der Ligationsprodukte aus (e),
   wobei die Amplifikation mittels der Polymerasekettenreaktion erfolgt und mindestens ein gegen die angefügten Linker gerichteter Primer sowie mindestens ein gegen die Sequenz des in Schritt (b) eingesetzten cDNA-Primers gerichteter Primer eingesetzt wird, und wobei die eingesetzten Primer gegebenenfalls an ihrem 3'-Ende um eine Extension von einer oder mehreren selektiven Basen verlängert sind,
(g) Auftrennen der Amplifikationsprodukte nach ihrer Länge, wobei die Amplifikationsprodukte an ihren Enden Sequenzen enthalten, an die die in Schritt (f) verwendeten Primer binden können, und
(h) Analysieren der Amplifikationsprodukte.

Vorzugsweise verwendet man bei diesem Verfahren degenerierte Zufallsprimer, die z. B. eine Länge von ca. 15 bis 25 Nudeotide aufweisen. Besonders bevorzugt besitzen diese degenerierten Zufallsprimer einen ausreichend hohen Degenerationsgrad, um statistisch so häufig wie ein 8 bis 12 Nucleotide langer, nicht-degenerierter Primer an eine DNA-Matrize zu binden. Der Degenerationsgrad der Zufallsprimer kann durch Verwendung von Oligonucleotidgemischen oder/und einen Einbau von degeneriert hybridisierenden Nucleotiden wie Inosin erreicht werden.

Eine Abwandlung des erfindungsgemäßen Verfahrens besteht darin, daß man während der ersten Amplifikationsrunde einen Primer verwendet, der gegen eine am 5'-Ende der mRNA bzw. am 3'-Ende der Erststrang-cDNA befestigte Ankersequenz gerichtet ist. Vorzugsweise wird bei dieser Abwandlung nach Entfernung der cap-Struktur in einer Einzelstrang-Ligation ein RNA-Oligonucleotid, das z. B. eine Länge von 20-30 Basen aufweist und vorzugsweise eine Schnittstelle für ein oder mehrere nachfolgend eingesetzte Restriktionsenzyme trägt, an das 5'-Ende der mRNA ligiert. Nach erfolgtem Restriktionsschritt der doppelsträngigen cDNA und erfolgter Linker-Ligation wird zur Reamplifikation dann ein Linkerprimer und ein von der Sequenz des cDNA-Primers oder von der Sequenz des besagten RNA-Oligonucleotids abgeleiteter Primer eingesetzt.

Ein derartiges Verfahren zur allgemeinen Analyse der Expression von Genen umfaßt vorzugsweise die Schritte:
(a) Isolieren von mRNA-Molekülen aus einer oder mehreren zur analysierenden Proben,
   (aa) Befestigen einer Ankersequenz am 5'-Ende der mRNA-Moleküle,
(b) Synthetisieren von cDNA-Erststrangmolekülen aus den mRNA-Molekülen,
(c) Amplifizieren der cDNA-Erststrangmoleküle und Markieren der Amplifikationsprodukte,
(d) transkriptspezifisches Verkürzen der Amplifikationsprodukte durch Spaltung mit einer oder mehreren Restriktionsendonucleasen,
(g) Auftrennen der Amplifikationsprodukte nach ihrer Länge und
(h) Analysieren der aufgetrennten Amplifikationsprodukte.

Alternativ wird ein weiteres bevorzugtes Verfahren zur allgemeinen Analyse der Expression von Genen bereitgestellt, gekennzeichnet durch die Schritte:
(a) Isolieren von mRNA-Molekülen aus einer oder mehreren Proben,
   (aa) Befestigen einer Ankersequenz am 5'-Ende der mRNA-Moleküle,
(b) Synthetisieren von cDNA-Erststrangmolekülen aus den mRNA-Molekülen,
(c) Amplifizieren der cDNA-Erststrangmoleküle,
(d) transkriptspezifisches Verkürzen der Amplifikationsprodukte durch Spaltung mit einer oder mehreren Restriktionsendonucleasen,
(e) Anfügen von Linkermolekülen an die Enden der Restriktionsfragmente,
(f) Reamplifizieren und Markieren der Restriktionsfragmente, wobei ein gegen das in Schritt (e) angefügte Linkermolekül gerichteter Primer und (i) ein gegen die in Schritt (aa) angefügte Ankersequenz gerichteter Primer oder (ii) ein gegen die in Schritt (b) eingesetzte cDNA-Primersequenz gerichteter Primer verwendet wird,
   (ff) gegebenenfalls nochmaliges Reamplifizieren und Markieren, wobei "nested"-Primer verwendet werden,
   (fff) gegebenenfalls subtraktives Hybridisieren von zwei miteinander zu vergleichenden Pools von Amplifikationsprodukten,
(g) Auftrennen der Amplifikationsprodukte nach ihrer Länge und
(h) Analysieren der aufgetrennten Amplifikationsprodukte.

Schritt (a) des erfindungsgemäßen Verfahrens umfaßt die Isolierung von mRNA-Molekülen aus zu analysierenden Proben, z. B. Gewebeproben, die aus Tieren, Pflanzen oder Mikroorganismen stammen können. Vorzugsweise geht man von mehreren, d. h. mindestens zwei Gewebeproben aus, um eine differenzielle Analyse der Genexpression aus den unterschiedlichen Gewebeproben durchführen zu können. Die Isolation der mRNA kann nach bekannten Verfahren (vgl. z. B. Sambrook et al, Molecular cloning: A Laboratory Manual, 2. Edition (1989), Cold Spring Harbor Laboratory Press, insbesondere Kapitel 7) erfolgen.

Schritt (aa) umfaßt das Befestigen einer ausgewählten Ankersequenz am 5'-Ende der RNA-Moleküle. Diese Ankersequenz umfaßt vorzugsweise ein RNA-Oligonucleotid und kann durch Decapping der mRNA und anschließende Einzelstrang-Ligation mittels T4-RNA-Ligase an den mRNA-Molekülen befestigt werden. Hierbei kann auch ein geeignetes modifiziertes Oligonucleotid, welches spezifisch an die cap-Struktur hybridisiert und eine Inkorporation der zu seiner eigenen Sequenz komplementären Sequenz im Schritt der reversen Transkription bewirkt, zum Einsatz kommen. Vorzugsweise enthält die Ankersequenz eine Schnittstelle für ein später zu verwendendes Restriktionsenzym, um die Erfassung jedes Transkripts auch bei Fehlen einer internen Schnittstelle sicherzustellen.

Schritt (b) des erfindungsgemäßen Verfahrens umfaßt die Synthese von Erststrang-cDNA-Molekülen aus mRNA. Hierzu wird vorzugsweise ein Oligo-dT-Primer an den Poly(A)-Schwanz der mRNA hybridisiert und mittels eines geeigneten Enzyms wie Reverse Transkriptase oder Tth-Polymerase verlängert. Der Oligo-dT-Primer kann am 5'-Ende oder/und am 3'-Ende Extensionen aufweisen. Die Extension am 5'-Ende kann beispielsweise eine Zufallssequenz mitvorzugsweise 20 bis 30 Nucleotiden, z. B. 24 Nucleotiden, sein. Die Extension am 3'-Ende kann eine Sequenz von einem oder mehreren Nucleotiden, vorzugsweise 1 bis 3 Nucleotiden, z. B. 1 oder 2 Nucleotiden, sein. Anstelle eines Oligo-dT-Primers können für die cDNA-Synthese auch Zufallsprimer, die vorzugsweise sehr kurz, z. B. 6 bis 10 Nucleotide lang sind, oder einen hohen Degenerationsgrad besitzen, um statistisch häufig genug an die mRNA zu binden, oder gegen eine bekannte Sequenz, gerichtete Oligonucleotide eingesetzt werden.

Schritt (c) des erfindungsgemäßen Verfahrens umfaßt die Synthese des cDNA-Zweitstrangs.

In Verbindung mit Schritt (c) kann auch eine Markierung der Amplifikationsprodukte durchgeführt werden. Hierzu kann einer oder beide der eingesetzten Primer markiert sein oder die Markierung kann durch Verwendung markierter Nucleotide erfolgen. Zur Markierung eignen sich einerseits radioaktive Markierungsgruppen, wie etwa ³²P, ³³P oder ³⁵S sowie Fluoreszenzfarbstoffe, Biotin oder Antigene, z. B. Digoxigenin. Die Markierung kann auch in einem separaten Schritt einer Primer-Verlängerung ("lineare PCR") mit den Amplifikations- bzw. Reamplifikationsprodukten als Template erfolgen.

Bei der Verfahrensvariante zur allgemeinen Genexpressionsanalyse erfolgt keine selektive Amplifizierung, sondern eine Amplifizierung aller vorhandenen cDNA-Erststrangmoleküle.

Hierzu wird vorzugsweise ein gegen die in Schritt (aa) angefügte Ankersequenz gerichteter Primer verwendet. Als Gegenprimer wird vorzugsweise ein von der Sequenz des in Schritt (b) verwendeten cDNA-Primers abgeleiteter Primer, z. B. ein gegen eine 5'-Extension des cDNA-Primers gerichteter Primer verwendet. Unter Verwendung dieser Primerkombination wird eine erste Amplifikationsrunde mit einigen, z. B. zehn Amplifikationszyklen, vorzugsweise unter "Long Range-PCR"-Bedingungen durchgeführt.

Schritt (d) des erfindungsgemäßen Verfahrens umfaßt die Transkript-spezifische Verkürzung der Amplifikationsprodukte durch Spaltung mit einem oder mehreren Restriktionsenzymen. Vorzugsweise wird ein Restriktionsenzym oder eine Kombination von Restriktionsenzymen verwendet, die das Amplifikationsprodukt mit einer statistisch hohen Wahrscheinlichkeit schneidet, vorzugsweise verwendet man Restriktionsenzyme mit vierbasiger Erkennungssequenz, wie etwa Mse I, Hinf I oder Sau3A I.

Wenn eine Wiedergewinnung der PCR-Produkte beabsichtigt ist, erfolgt als Schritt (e) eine Ligation von doppelsträngigen Linker- bzw. Adaptermolekülen an die Enden der in Schritt (d) erzeugten Restriktionsfragmente. Vorzugsweise werden Linkermoleküle mit unterschiedlichen Enden verwendet, wobei ein Ende des Linkers auf die durch das Restriktionsenzym erzeugten Enden der Restriktionsfragmente paßt, während das andere Ende, z. B. durch einen geeigneten einzelsträngigen Überhang, so gestaltet ist, daß keine Ligation (weder Selbst- noch Fremdligation) möglich ist. Weiterhin können die Enden der Restriktionsfragmente durch nachfolgende Modifikation, z. B. Auffüllung überhängender Enden, an die Enden der jeweils verwendeten Linkermoleküle angepaßt werden.

Schritt (f) des erfindungsgemäßen Verfahrens umfaßt das Reamplifizieren und - sofern dies noch nicht erfolgt ist - Markieren der Restriktionsfragmente. Hierbei wird vorzugsweise eine Primerkombination verwendet, umfassend (i) einen gegen das Linkermolekül gerichteten Primer und (ii) einen gegen die Sequenz des in Schritt (aa) an das 5'-Ende der mRNA angefügten Ankeroligonucleotids gerichteten Primer oder einen gegen die Sequenz des in Schritt (b) eingesetzten cDNA-Primers gerichteten Primer. Alle eingesetzten Primer können mit den zuvor in Schrit (c) verwendeten Primern identisch sein oder eine Extension von einer oder mehreren zusätzlichen Basen an ihrem 3'-Ende aufweisen. Die Markierung der Reamplifikationsprodukte erfolgt wie bei Schritt (c) beschrieben.

Werden fluoreszenzmarkierte Primer eingesetzt, so kann die Fluoreszenzfarbe zur Codierung des jeweiligen Primers verwendet werden; beispielsweise kann die letzte Base eines an seinem 3'-Ende um eine Base verlängerten Primers durch eine von mehreren, z. B. vier Farben gekennzeichnet werden. Werden in Schritt (c) Biotin-markierte Primer eingesetzt, so kann vor der Reamplifikation eine Reinigung der Amplifikationsprodukte durch Bindung an Streptavidin-beschichtete magnetische Partikel oder Streptavidin-beschichtete Reaktionsgefäße vorgenommen werden.

Um eine Unterteilung der in diesem Schritt (bei Verwendung von Familien-unspezifischen Primern) erhaltenen ca. 15000 verschiedenen PCR-Produkte zu ermöglichen, tragen die PCR-Primer an ihrem 3'-Ende vorzugsweise eine Extension. Geeignet ist beispielsweise eine zweibasige Extension am Linkerprimer, wobei 16 verschiedene Kombinationen möglich sind und eine einbasige Extension am Gegenprimer (A, C oder G, wobei drei verschiedene Kombinationen möglich sind), so daß pro zu untersuchender Probe 3 x 16 = 48 Amplifikationsreaktionen mit unterschiedlichen Primerkombinationen durchgeführt werden müssen. Weiterhin ist es alternativ zu diesem "3'-Ansatz" möglich, die Amplifikation am 5'-Ende durchzuführen, indem Linker-Primer und ein gegen die Ankersequenz gerichteter Primer kombiniert werden. Diese Verfahrensvariante hat ein Vorteil, daß man ohne einen Primer auskommt, der eine Oligo(dT) Sequenz enthält.

Der fakultative Schritt (ff) des erfindungsgemäßen Verfahrens umfaßt eine Reamplifikation der in Schritt (f) erhaltenen Amplifikationsprodukte unter Verwendung von "nested"-Primern, d. h. mit Primern, die an ihrem 3'-Ende gegenüber den in Schritt (f) verwendeten Primern um eine oder mehrere Basen verlängert sind. Durch diese nochmalige Reamplifikation können die in geringerer Konzentration vorhandenen "seltenen" Amplifikationsprodukte bis zu einer detektierbaren Konzentation amplifiziert und anschließend nachgewiesen werden (McClelland und Welsh (1994), PCR Methods Appl. 4, 66-81; Ralph et al. (1993), Proc. Natl. Acad. Sci. USA 90, 10710-10714).

Der fakultative Schritt (fff) des erfindungsgemäßen Verfahrens umfaßt eine subtraktive Hybridisierung zweier zu vergleichender Amplifikationsproduktpools, um nachfolgend nur solche Produkte gelelektrophoretisch zu untersuchen, deren Häufigkeit sich zwischen den beiden Pools unterscheidet. Diese Produkte repräsentieren dann in den beiden Ausgangsproben differenziell exprimierte Gene und sind daher von besonderem Interesse, während in vielen Fällen auf die Kenntnis nicht-differenziell exprimierter Gene verzichtet werden kann. Die Methode der subtraktiven Hybridisierung ist beispielsweise bei Bautz und Reilly (1966), Science 151, 328-330 und Zimmermann et al. (1980), Cell 21, 709-715 beschrieben. Zur Durchführung einer derartigen subtraktiven Hybridisierung kann beispielsweise einer der zu vergleichenden Pools von Amplifikationsprodukten ("Driver") markiert, z. B. biotinyliert werden, z. B. durch Verwendung biotinylierter Primer und dann mit einem Unterschuß des anderen Pools ("Tester") hybridisiert werden. Anschließend können die markierten Hybridisierungsprodukte, z. B. bei einer Biotinmarkierung, durch Bindung an Streptavidin-beschichtete Magnetpartikel abgetrennt werden (vgl. z. B. Wang und Brown (1991), Proc. Nat. Acad. Sci. USA 88, 11505-11509). Sofern erforderlich, kann dieser Subtraktionsschritt auch mehrfach hintereinander durchgeführt werden. Die in Lösung verbleibenden Amplifikationsprodukte können dann erneut einer Amplifikation unterzogen werden, um für den anschließenden Nachweis ausreichende Mengen an DNA zur Verfügung zu haben. Wird nach der Subtraktion eine solche Amplifikation durchgeführt, so wird vorzugsweise eine zum Nachweis geeignete Markierungsgruppe erst in diesem Schritt eingeführt. Auf diese Weise kann man z. B. eine Biotinmarkierung zur Abtrennung von Hybriden nach der Subtraktion von einer Markierung zur Visualisierung von Fragmenten unterscheiden.

Diese Variante des erfindungsgemäßen Verfahrens läßt sich sowohl zum Nachweis von "Ja"- bzw. "Nein"-Expressionszuständen anwenden (hoher Überschuß von markierter DNA, wenige Hybridisierungsrunden) als auch zum Nachweis der Herauf- bzw. Herabregulation von Genen (geringer Überschuß an markierter DNA, mehr Hybridisierungsrunden) nutzen (Fargnoli et al. (1990), Analyt. Biochem. 187, 364-373).

An die Subtraktion kann sich gegebenenfalls auch eine Normalisierung, d. h. eine Nivellierung der verschiedenen Konzentrationen der einzelnen Amplifikationsprodukte, anschließen. Diese Normalisierung stellt sicher, daß im nachfolgenden Schritt (h), der Analyse der aufgetrennten Amplifikationsprodukte, auch seltene Transkripte repräsentierende, in geringer Konzentration vorliegende Amplifikationsprodukte erkannt werden. Eine solche Normalisierung kann vor, nach oder während der sich an die Subtraktion anschließenden Amplifikation vorgenommen werden. Ein geeignetes Verfahren zur Normalisierung ist beispielsweise bei Ko (1990) Nucleic Acids Res. 19, 5705-5711, beschrieben.

Schritt (g) des erfindungsgemäßen Verfahrens umfaßt die Auftrennung der Amplifikations- bzw. Reamplifikationsprodukte nach ihrer Länge in einem geeigneten System, z. B. elektrophoretisch oder durch HPLC. Besonders bevorzugt ist eine Elektrophorese in einem denaturierenden oder nicht-denaturierenden Polyacrylamidgel. Diese Auftrennung kann mit einem einzelnen Reaktionsansatz oder parallel mit mehreren, miteinander zu vergleichenden Reaktionsansätzen erfolgen. Die Visualisierung der aufgetrennten Produkte erfolgt gemäß der eingeführten Markierungsgruppe, z. B. durch Autoradiographie, Chemilumineszenz oder spezifischen Nachweis markierter Moleküle durch Anfärbung oder Bestrahlung mit UV-Licht. Der Nachweis der Produkte kann im Gel (unbehandelt oder fixiert und getrocknet) oder nach Übertragung auf einen geeigneten Träger, vorzugsweise eine Hybridisierungsmembran, erfolgen. Besonders geeignet für eine solche Übertragung ist das Verfahren der Direct Blotting-Elektrophorese. Der Nachweis kann auch durch unspezifische DNA-Färbung mittels Silberverbindungen oder geeigneter Farbstoffe (z. B. SYBR Green I) oder durch Hybridisierung der die Produkte tragenden Membran mit einer geeigneten Sonde erfolgen. Beim Einsatz von Fluoreszenzgruppen zur Markierung können Auftrennung und Nachweis mittels einer automatisierten Sequenzierapparatur oder/und der Nachweis mittels eines Fluoreszenz-Gelscanners bzw. Fluorimagers erfolgen.

Schritt (h) umfaßt die Analyse der aufgetrennten Amplifikations- bzw. Reamplifikationsprodukte. Hierbei können interessierende Banden isoliert und erneut amplifiziert werden. Bei einer erneuten Amplifizierung können die gleichen Primer wie bei der Reamplifizierung gemäß Schritt (f) verwendet werden. Die Isolierung der Banden kann z. B. durch Ausschneiden mittels eines Skalpells aus dem Gel bzw. aus dem Träger erfolgen, auf den die aufgetrennten Produkte übertragen wurden. Die ausgeschnittenen Banden können anschließend in einem PCR-Ansatz zur Reamplifikation inkubiert werden. Die dabei erhaltenen Produkte können ohne Klonierungsschritt zur Direktsequenzierung eingesetzt werden. Hierfür ist das Cycle-Sequencing-Verfahren besonders geeignet. Als Sequenzierprimer können die zuvor verwendeten PCR-Primer eingesetzt werden. Alternativ können zur Reamplifikation oder/und zur Sequenzierung an ihrem 3'-Ende verlängerte Primer eingesetzt werden, um mögliche Verunreinigungen einer Bande durch andere Amplifikationsprodukte auszublenden. Die notwendige 3'-Verlängerung der hierzu verwendeten Primer läßt sich etwa erschließen, wenn die Detektion der Banden über Fluoreszenz-markierte Primer erfolgt und in Schritt (f) bzw. (ff) Mischungen verschiedener Fluoreszenzmarkierter Primer, deren letzte Base oder Basen durch ihre jeweilige Markierung codiert sind, eingesetzt worden sind.

Vor dem Einsatz zur Sequenzierung können die Reamplifikationsprodukte, vorzugsweise durch nochmalige Elektrophorese in einem denaturierenden Polyacrylamidgel, auf ihre Reinheit überprüft werden. Treten noch Amplifikationsprodukte auf, die sich in ihrer Größe vom gewünschten Amplifikationsprodukt unterscheiden, so kann eine weitere Reinigung durch erneutes Ausschneiden der gewünschten Bande und nachfolgende Reamplifikation erfolgen. Die Reamplifikationsprodukte können weiterhin auch in Southern-, Northern- oder in situ-Hybridisierungsexperimenten eingesetzt werden. Außerdem können sie - sofern sie eine noch unbekannte Nucleinsäuresequenz aufweisen - zur Isolierung zugehöriger genomischer bzw. cDNA-Klone aus entsprechenden Banken verwendet werden.

Ist die Zahl an verschiedenen Produkten, die bei einer Amplifikationsreaktion erhalten werden, hinreichend klein, so kann die Reamplifikation einzelner Produkte direkt aus der Gesamtreaktion erfolgen, indem geeignete selektive Primer eingesetzt werden, die beispielsweise gegenüber den zuvor eingesetzten Primern an ihrem 3'-Ende eine Extension von mindestens einer Base haben. Eine solche Situation wird insbesondere dann auftreten, wenn gemäß Schritt (fff) eine Subtraktion in mehreren parallelen Ansätzen durchgeführt worden ist. Jede Subtraktionsreaktion wird dann nur noch wenige, beispielsweise 5-10 verschiedene Amplifikationsprodukte enthalten. Um selektiv jedes hiervon einzeln durch Amplifikation aus der Subtrak tionsreaktion gewinnen zu können, ist die Kenntnis einiger an die zuvor eingesetzten Amplifikationsprimer grenzender Basen hilfreich. Diese Kenntnis kann beispielsweise dadurch erhalten werden, daß für die nach der Subtraktion erfolgende Amplifikation Mischungen von jeweils vier Primern eingesetzt werden, die an ihrem 3'-Ende um eine der vier möglichen Basen verlängert sind und die eine Fluoreszenzmarkierung tragen, deren Farbe eindeutig die jeweils letzte Base eines Primers codiert. Werden die Amplifikationsprodukte nun aufgetrennt und im UV-Licht untersucht, so läßt die Fluoreszenzfarbe einer Bande Rückschlüsse auf den im entsprechenden Produkt inkorporierten Primer zu, welcher dann nachfolgend zur selektiven Reamplifikation eingesetztwerden kann. Um eine ausreichende Zahl von Codierungsmöglichkeiten zu erhalten (beispielsweise 4¹, 4² = 16, 4³ = 64 etc.), können in mehreren parallelen Ansätzen geeignete fluoreszenzcodierte Primer mit jeweils unmarkierten Gegenprimern kombiniert und zur Amplifikation eingesetzt werden.

Weiterhin ist eine Reihe von Modifikationen für das erfindungsgemäße Verfahren denkbar. So kann man in Schritt (b) die Erststrang-cDNA z. B. an ihrem 3'-Ende mit einer spezifischen Nucleinsäuresequenz versehen und bei der nachfolgenden Amplifikation in Schritt (c) ein gegen diese spezifische Nucleinsäuresequenz gerichtetes Oligonucleotid verwenden. Die spezifische Nucleinsäuresequenz umfaßt beispielsweise ein Homopolymer oder eine Zufallssequenz mit einer Länge von vorzugsweise 20 bis 30 Nucleotiden.

Eine weitere Modifikation des erfindungsgemäßen Verfahrens könnte beispielsweise darin bestehen, daß die Erststrang-cDNA durch Selbstligation, z. B. in einem Ligationsansatz mit RNA-Ligase, in oligomere oder/und zirkuläre Moleküle überführt wird und daß für die nachfolgende Amplifikation in Schritt (c) Gegenprimer ein zumindest teilweise zum cDNA-Primer komplementäres, "down-stream" gerichtetes Oligonucleotid verwendet wird, das an seinem 3'-Ende gegebenenfalls eine Extension von vorzugsweise 1 bis 3 Nucleotiden aufweist.

Noch eine weitere Modifikation des erfindungsgemäßen Verfahrens besteht darin, den Schritt (d) der Restriktionsspaltung bereits ohne vorherige Amplifikation an doppelsträngiger cDNA durchzuführen. Durch diese Vorgehensweise kann der Verlust sehr langer Transkripte von mehreren kb Länge vermieden werden, die bei einer Amplifikation der vollständigen Erststrang-cDNA verloren gehen könnten.

Weiterhin ist bevorzugt, daß die im erfindungsgemäßen Verfahren und in den weiter unten diskutierten Abwandlungen dieses Verfahrens eingesetzten Oligonucleotidprimer eine Länge von vorzugsweise mindestens 15 Nucleotiden, besonders bevorzugt von mindestens 18 Nucleotiden aufweisen, um die Reproduzierbarkeit der erhaltenen Bandenmuster zu verbessern. Weiterhin können die Oligonucleotidprimer auch durch synthetische Nucleinsäureanaloga oder -derivate ersetzt werden, z. B. durch peptidische Nucleinsäuren (PNA), vorausgesetzt, daß sie an ihrem 3'-Ende eine von der Polymerase akzeptierte 3'-OH-Gruppe aufweisen.

Darüber hinaus können die im erfindungsgemäßen Verfahren und dessen Abwandlungen eingesetzten Oligonucleotide, d. h. Ankeroligonucleotide, cDNA-Primer, Amplifikationsprimer etc. eine oder mehrere Erkennungs- bzw. Schnittstellen für Restriktionsenzyme enthalten. Auf diese Weise läßt sich der Verlust von nicht durch das Restriktionsenzym geschnittenen Amplifikationsprodukten vermeiden, wenn vor der Linkerligation keine Auffüllung der Enden durchgeführt wird.

Das erfindungsgemäße Verfahren besitzt gegenüber der bekannten Differential Display-Technik überraschende Vorteile, da hier perfekt passende Primer in einer für Amplifikationsreaktionen optimalen Länge zum Einsatz kommen können. Dies führt zu einer hohen Reproduzierbarkeit des Verfahrens. Hingegen wird bei den DD-Experimenten des Standes der Technik mit den dort verwendeten, oftmals sehr kurzen und unter Tolerierung von Fehlpaarungen bindenden Primern (Bertioli et al., Supra) nur eine unzureichende Reproduzierbarkeit erhalten, da das Verfahren extrem von bestimmten Verfahrensparametern, z. B. der Annealing-Temperatur während der PCR abhängig ist. Diese bei DDRT-Experimenten zu beobachtende schlechte Reproduzierbarkeit hat zur Folge, daß pro Reaktion mindestens ein oder zwei Replikaexperimente durchgeführt werden müssen, um Artefakte als solche erkennen zu können. Zusammen mit der hohen Anzahl an verschiedenen Primer-Kombinationen, die erforderlich sind, um mit einer gewissen Wahrscheinlichkeit zumindest einen Großteil aller Transkripte erfassen zu können (Bauer et al., Supra), wird die Durchführung der DDRT des Standes der Technik sehr aufwendig. Trotzdem gibt es dort aufgrund des statistischen Bindeverhaltens von Zufallsprimern keine definierte Zahl an Primerkombinationen, die die Erfassung aller Transkripte garantieren könnte. Beim erfindungsgemäßen Verfahren hingegen sind Replikaexperimente überflüssig, und die Zahl für eine vollständige Untersuchung erforderlicher Primerkombinationen läßt sich aus der erwarteten Zahl an Transkripten in einem untersuchten Gewebe (beispielsweise 15000) und der Zahl an Produkten, die in einer einzelnen Amplifikationsreaktion zugelassen werden sollen (beispielsweise 100), leicht errechnen. Dabei kann die Erfassung eines jeden Transkripts sichergestellt werden, indem die als cDNA-Primer bzw. als Anker eingesetzten Oligonucleotide Schnittstellen des in Schritt (d) verwendeten Restriktionsenzyms enthalten. Darüber hinaus ermöglicht der Verzicht auf Replikareaktionen die simultane Untersuchung von 30 oder mehr verschiedenen RNA-Proben auf einem einzigen Gel.

Ein weiterer Vorteil des erfindungsgemäßen Verfahrens gegenüber der DD-Technik besteht darin, daß die differenzielle Expression aller untersuchten Banden durch Northern-Experimente bestätigt werden konnte. Bei den DD-Experimenten des Standes der Technik konnten im Gegensatz dazu beispielsweise von insgesamt 15 erhaltenen Banden nur fünf als differenziell exprimiert bestätigt werden, während vier ein falsch positives Signal zeigten und weitere sechs im Northern-Blot überhaupt kein Signal ergaben (vgl. Liang et al, Supra).

Weiterhin schließt das erfindungsgemäße Verfahren die mehrfache Erfassung eines Transkripts aus. Dies resultiert aus der Tatsache, daß bei Verwendung nicht-phosphorylierter Oligonucleotide für die Linkerpräparation eine Amplifikation immer nur zwischen einem definierten Bereich eines Transkripts (seinem 3'-Ende, seinem 5'-Ende oder einer Familien-spezifischen Domäne) und einem Linker, aber niemals zwischen zwei anligierten Linkem stattfinden kann. In DDRT-Experimenten des Standes der Technik hingegen kann ein gegebenes Transkript von verschiedenen Primerkombinationen erkannt und auf diese Weise mehrfach detektiert werden. So wurde beispielsweise ein und dasselbe Transkript durch 15 verschiedene DDRT-Banden repräsentiert, die durch Kombination verschiedener 5'-Primer mit verschiedenen 3'-Primern generiert worden waren (Linskens et al. (1995), Nucleic Acids Res. 23, 3244-3251).

Darüber hinaus können die beim erfindungsgemäßen Verfahren erhaltenen Produkte auf einfache Weise direkt nach der Reamplifikation sequenziert werden, ohne daß ein Klonierungsschritt erforderlich ist. Diese Sequenzierung erfolgt vorzugsweise durch die Cycle-Sequenzierungstechnik. Diese Technik ist für die bei DD-Experimenten verwendeten Primer mit einer Länge von etwa 10 Nucleotiden ungeeignet. Zudem sind DDRT-Produkte des Standes der Technik oftmals von zwei Molekülen desselben Primers flankiert, was die Verwendung des jeweiligen Amplifikationsprimers als Sequenzierprimer ausschließt (Guimaraes et al. (1995), Nucleic Acids Res. 23, 1832-1833).

Ein weiterer Vorteil des erfindungsgemäßen Verfahrens ist, sofern der Schritt (fff) einer subtraktiven Hybridisierung vorgenommen wird, eine starke Verringerung der Zahl der durchzuführenden Einzelreaktionen sowie der zu untersuchenden Amplifikationsprodukte. Während zur Durchführung von DD-Experimenten des Standes der Technik mehrere hundert PCR-Ansätze erforderlich sind, die anschließend in mehreren hundert Polyacrylamidgel-Spuren aufgetrennt werden (Liang und Pardee, Supra; Bauer et al., Supra), ist es beim erfindungsgemäßen Verfahren möglich, die in Schritt (b) generierten cDNA-Pools durch Verwendung von Primern in Schritt (f), die an ihrem 3'-Ende gegenüber der Sequenz ihrer gemeinsamen Bindungsstelle um jeweils eine oder zwei Basen verlängert sind, in mehrere, beispielsweise acht oder 12 Subpools zu unterteilen. Einander entsprechende Subpools werden dann parallel subtrahiert und die Produkte einer jeden Subtraktion in einer eigenen Gelspur analysiert.

Gegenüber dem bekannten Verfahren des "cDNA-RDA" (Representational Difference Analysis of cDNA; Hubank und Schatz (1994), Nucleic Acids Res. 22, 5640-5648) hat das erfindungsgemäße Verfahren mehrere Vorteile: erstens wird jedes Transkript hier durch genau ein Amplifikationsprodukt repräsentiert, was sowohl den ungewollten Verlust mancher Transkripte als auch die mehrfache Detektion ein und desselben Transkripts vermeidet, zweitens führt die gegenüber cDNA-RDA wie gegenüber der subtraktiven Hybridisierung vollständiger cDNA-Moleküle verringerte Komplexität der Hybridisierungsreaktionen zu einem vollständigeren und schnelleren Ablauf der Reaktionen (Sargent (1987), Methods in Enzymol. 152, 423-432) und drittens erlauben die im Rahmen des erfindungsgemäßen Verfahrens vorgesehenen Schritte die gezielte Detektion seltener Transkripte.

Weiterhin wird die Sequenzierung von DD-Produkten durch die oft gleichzeitige Anwesenheit von kontaminierenden cDNA-Molekülen mit gleicher Größe behindert. Aus diesen Gründen ist für DD-Produkte eine aufwendige Reinigungsprozedur zur weiteren Analyse erforderlich (Liang et Pardee, Supra; Bauer et al, Supra; Li et l (1994), Nucleic Acids Res. 22, 1764-1765).

Darüber hinaus erlaubt das erfindungsgemäße Verfahren im Gegensatz zur DD-Methode des Standes der Technik eine zumindest semiquantitative Analyse der Resultate in bezug auf die Signalstärke eines gegebenen Transkripts. Dies zeigt ein Vergleich des erfindungsgemäßen Verfahrens mit zur Kontrolle durchgeführten Northem-Hybridisierungen.

Aufgrund seiner Sensitivität kann das erfindungsgemäße Verfahren auch eingesetzt werden, um interessierende Transkripte aus wenigen oder sogar einzelnen Zellen zu analysieren. Daher kann diese Technik, beispielsweise in der Entwicklungsbiologie zur Isolation neuer Gene, zur Identifizierung neuer Mitglieder interessierender Genfamilien, zur genauen Definition unterschiedlicher Lebensstadien eines Organismus und zur Untersuchung des Lebenszyklus einer Zelle und damit auch zur Untersuchung von Abweichungen gegenüber dem normalen Lebenszyklus, z. B. bei der Krebsforschung, wie bereits in Kotsinas et al., (Int. J. Oncology 315 (1993),841-855) beschrieben, eingesetzt werden.

Ein weiterer Gegenstand der vorliegenden Erfindung sind Abwandlungen des oben beschriebenen Verfahrens. Eine dieser Abwandlungen besteht darin, daß in der Ausführungsform zur allgemeinen Expressionsanalyse die cDNA-Zweitstrangsynthese auf eine andere gängige Weise erfolgt. Beispielsweise kann die Neigung einzelsträngiger cDNA durch Ausbildung terminaler Haarnadelschleifen ausgenutzt werden, oder es kann eine spezifische Nudeinsäuresequenz, beispielsweise eine Homopolymer oder ein einzelsträngiges Oligonucleotid, an die Erststrang-cDNA angefügt werden, um eine Primer-Bindungsstelle für die Zweitstrangsynthese bereitzustellen (siehe beispielsweise Sambrook et al., Supra, insbesondere Kap. 8; Troutt et al. (1992), Proc. Natl. Acad. Sci. USA 89, 9823-9825).

Noch eine weitere Abwandlung des beschriebenen Verfahrens besteht darin, daß man die Erststrang-cDNA, wie oben beschrieben, mit einer spezifischen Nucleinsäuresequenz versieht und bei der Amplifikation im Schritt (c) als ersten Primer ein gegen die angefügte spezifische Nucleinsäuresequenz gerichtetes Oligonucleotid und ein von der Sequenz des cDNA-Primers abgeleitetes Oligonucleotid als Gegenprimer verwendet. Der Gegenprimer kann gegebenenfalls eine 3'-Extension aufweisen. Die spezifische Nudeinsäuresequenz kann ein Homopolymer oder eine Zufallssequenz umfassen. Die spezifische Nudeinsäuresequenz wird vorzugsweise durch Ligation, z. B. mittels RNA-Ligase, an die Erststrang-cDNA ligiert. Sie kann an ihrem 3'-Ende modifiziert sein (z. B. durch Einführung einer Aminogruppe), um die Ligation von mehr als einem Oligonucleotidmolekül an die cDNA zu unterbinden.

In einer noch weiteren Abwandlung des erfindungsgemäßen Verfahrens wird die Erststrang-cDNA durch Selbstligation, z. B. mit RNA-Ligase, in oligomere oder/und zirkuläre Moleküle überführt und in der nachfolgenden Amplifikation in Schritt (c) werden als Primer zwei gegen den cDNA-Primer gerichtete Oligonucleotide verwendet, von denen einer "upstream" und der andere "downstream" gerichtet ist. Die Reamplifikation nach dem Restriktions- und Ligationsschritt kann neben dem gegen den Linker gerichteten Primer mit dem "upstream" oder/und mit dem "down-stream"-Primer erfolgen. Jeder der beiden Primer kann an seinem 3'-Ende eine Extension aufweisen, d. h. um eine oder mehrere Basen verlängert sein. Vorzugsweise werden die "upstream"- und "downstream"-Primer so ausgewählt, daß sie nicht oder nur wenig überlappen, um eine Hybridisierung beider Primer während der Amplifikation zu vermeiden.

Gegenüber bekannten, auf subtraktiver Hybridisierung beruhenden Verfahren zur differenziellen Expressionsanalyse von Genen zeichnet sich das erfindungsgemäße Verfahren durch verbesserte Reproduzierbarkeit aus und kann daher routinemäßig eingesetzt werden. Diese verbesserte Reproduzierbarkeit beruht darauf, daß im Stand der Technik cDNA-Moleküle mit einer Länge von oft bis zu mehreren Tausend bp eingesetzt und darüber hinaus komplette mRNA- bzw. cDNA-Pools verwendet wurden. Diese Probleme treten nicht auf, wenn die subtraktive Hybridisierung zur Analyse von Amplifikationsprodukten verwendet wird, die durch das erfindungsgemäße Verfahren gewonnen wurden. Die Amplifikationsprodukte sind nämlich gegenüber den cDNA-Molekülen des Standes der Technik stark verkürzt und durch das Design der Primer (z. B. die Auswahl verschiedener 3'-Extensionen für ansonsten identische Primer) läßt sich ein Pool von Amplifikationsprodukten völlig problemlos in mehrere, z. B. 8, 12 oder 16 Subpools unterteilen. Einander entsprechende Subpools können dann parallel subtrahiert werden.

Noch ein weiterer Gegenstand der vorliegenden Erfindung ist ein Reagenzienkit zur Analyse der Expression von Genen, insbesondere zur Anwendung in einem erfindungsgemäßen Verfahren. Dieser Reagenzienkit umfaßt vorzugsweise in räumlich getrennter Anordnung:
(a) ein Enzym zur cDNA-Erststrangsynthese,
(b) mindestens ein Enzym zur Synthese von cDNA-Zweitsträngen und zur Amplifikation von DNA-Fragmenten,
(c) gegebenenfalls mindestens ein Restriktionsenzym,
(d) gegebenenfalls mindestens ein doppelsträngiges DNA-Linkermolekül und Mittel zur Befestigung des Linkermoleküls an DNA-Fragmenten,
(e) gegebenenfalls mindestens ein einzelsträngiges RNA-Oligonucleotid und Mittel zur Befestigung des RNA-Oligonudeotids am 5'-Ende von mRNA-Molekülen,
(f) einzelsträngige Nucleinsäure-Primermoleküle ausgewählt aus der Gruppe, umfassend (i) mindestens ein Oligo-dT-Nucleotid, (ii) Primer gemäß (iii) mit einer 3'-Extension, oder/und 5'-Extension, wobei einer der Primer gemäß (i-ii) eine Markierungsgruppe ausgewählt aus Fluoreszenzfarbstoffen, Biotin oder Antigenen träger und (iii) Primer gemäß (i) oder (ii), die eine durch die Art ihrer Markierung, z. B. die Farbe einer Fluoreszenzmarkierung codierte 3'-Extension enthalten,
(g) Mittel zur Markierung und zum Nachweis von Nucleinsäuren und
(h) gegebenenfalls weitere Reagenzien wie RNase-Inhibitor, Nucleotide, Puffer, Streptavidin-beschichtete Reaktionsgefäße oder magnetische Partikel.

Einzelne Reagenzien des Kits können unter Umständen auch separat bezogen werden. Neben den Reagenzien kann der Kit noch geeignete Puffer für die jeweils verwendeten Enzyme enthalten.

Weiterhin soll die vorliegende Erfindung durch die folgenden Figuren, Sequenzprotokolle und Beispiele erläutert werden.

Es zeigen:

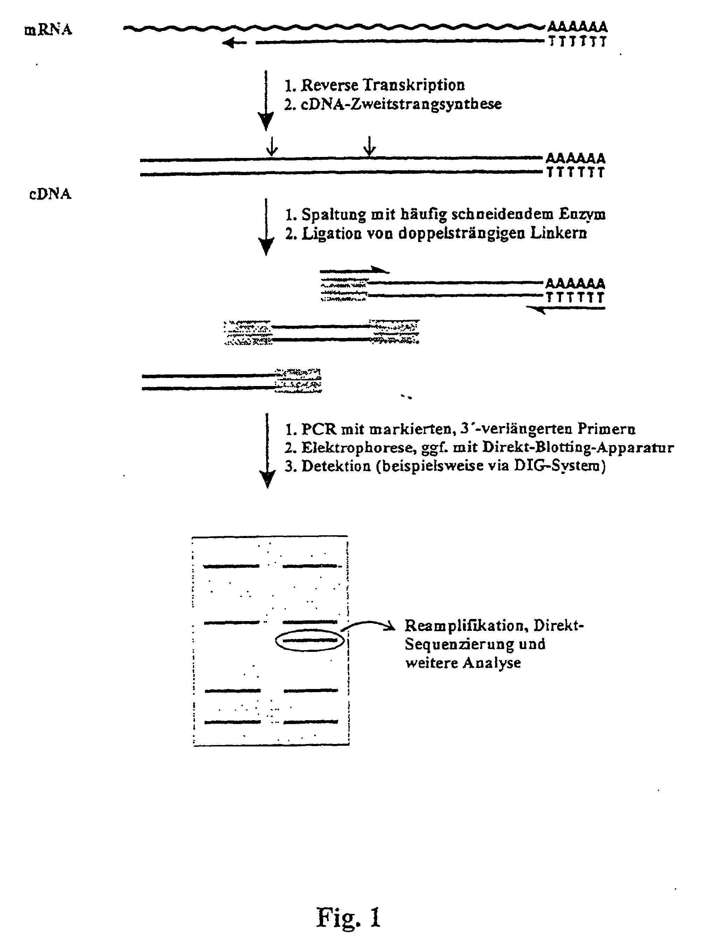

- Fig. 1: eine schematische Darstellung einer Ausführungsform des erfindungsgemäßen Verfahrens zur allgemeinen Expressionsanalyse von Genen;

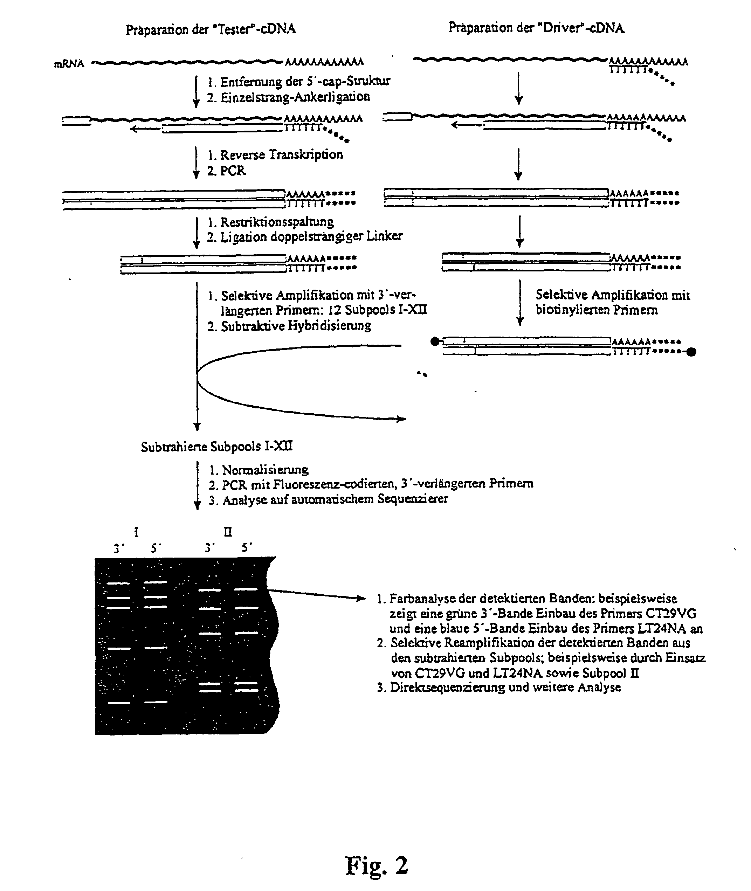

- Fig. 2: eine schematische Darstellung einer weiteren Ausführungsform des erfindungsgemäßen Verfahrens zur allgemeinen Expressionsanalyse von Genen unter Einbeziehung eines Subtraktionsschritts und unter Einsatz von fluoreszenzcodierten Primem zur Reamplifikation;
- Fig. 3: eine schematische Darstellung einer weiteren Ausführungsform des erfindungsgemäßen Verfahrens zur Expressionsanalyse von Genfamilien;

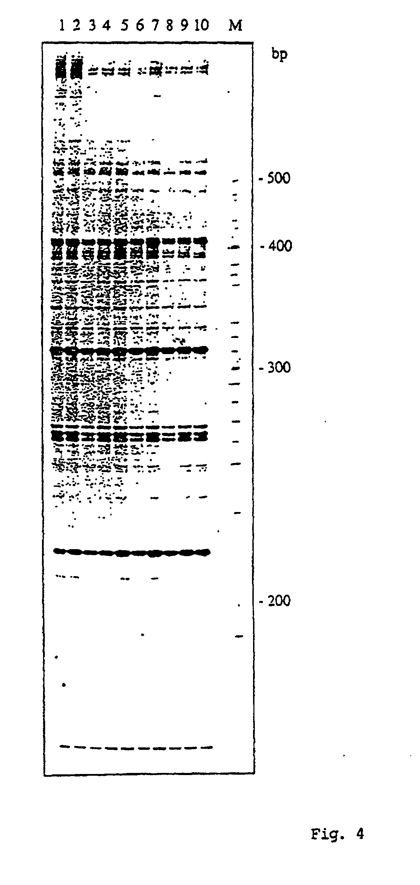

- Fig. 4: die Reproduzierbarkeit des Verfahrens, demonstriert durch zehn unabhängige Experimente;
- Fig. 5: das Ergebnis der Isolierung differenziell exprimierter MADS-Box-Gene unter Verwendung des erfindungsgemäßen Verfahrens,
- Fig. 6: die Zuordnung der durch das erfindungsgemäße Verfahren identifizierten Gene zu MADS-Box-Gensubfamilien.

- SEQ ID No. 1: die Sequenz eines RNA-Oligonucleotids,
- SEQ ID No. 2: die Sequenz des Primers CT29V,
- SEQ ID No. 3: die Sequenz des Primers AP23,
- SEQ ID No. 4: die Sequenz des Primers LT20,
- SEQ ID No. 5: die Sequenz des Primers LT24,
- SEQ ID No. 6: die Sequenz des Primers P038,
- SEQ ID No. 7: die Sequenz des Primers P041,
- SEQ ID No. 8: die Sequenz des Primers P042,
- SEQ ID No. 9: die Sequenz des Primers P043,
- SEQ ID No. 10: die Sequenz des Primers LR32,
- SEQ ID No. 11: die Sequenz des Primers BLT18,
- SEQ ID No. 12: die Sequenz des Primers P046,
- SEQ ID No. 13: die Sequenz des Primers P009 und
- SEQ ID No. 14: die MADS-Domänen-Konsensus-Sequenz.

### Beispiel 1:

### Gewinnung von RNA

Maispflanzen (Zea mays ssp. mays), Inzuchtlinie T232, und Teosintepflanzen (Zea mays ssp. parviglumis) Rasse Central Balsas, wurden für 7-8 Wochen im Treibhaus kultiviert. Zu diesem Zeitpunkt weisen die Pflanzen die von Cheng et al. (Am. J. Bot. 70 (1983), 450-462) definierten Entwicklungsstadien I und J auf.

Männliche und weibliche Infloreszenzen wurden geerntet, in flüssigem Stickstoff eingefroren und zur Gewinnung von Gesamt-RNA gemäß der Guanidiniumchlorid-Isolationsmethode nach Sambrook et al (Molecular Cloning: A Laboratory Manual, 2. Edition (1989), Cold Spring Harbor Laboratory Press) verwendet.

Die Qualität der RNA-Präparationen wurde durch Northern Hybridisierung (Sambrook et al, Supra) mit einer Mais-Glycerinaldehyd-3-phosphat-Dehydrogenase (GAPDH)-Sonde getestet. Zur Entfernung von genomischer DNA wurden 50 µg Gesamt-RNA mit 1 µl des RNase-Inhibitors RNasin (Promega, 40 U/µl) und 5 µl RQ1 RNase-freier DNase (Promega, 1 U/µl) in 100 µl Reverse Transkriptase (RT)-Puffer (Frohman et al, Proc. Natl. Acad. Sci. USA 85 (1988), 8998-9002) für 15 min. bei 37 °C inkubiert. Die Proben wurden mit Tris/EDTA (TE) gesättigtem Phenol und mit Chloroform/Isoamylalkohol (24:1) extrahiert und mit Ethanol gefällt. Nach Zentrifugation für 30 min. bei 20.000 x g und 0 °C wurde die Gesamt-RNA in 1 mM EDTA (pH 7,5) gelöst. Die Poly(A)⁺ mRNA wurde unter Verwendung von oligo (dT)-Dynabeads (Dynal, Oslo) isoliert.

### Beispiel 2:

### Analyse geschlechtsabhängiger Genexpression

Die in Beispiel 1 aus männlichen und weiblichen Maisinfloreszenzen isolierte Gesamt-RNA wurde zunächst dephosphoryliert. 50 µg gefällte RNA wurden hierzu in einer Mischung aus 40 µl mit Diethylpyrocarbonat behandeltem Wasser ("DEPC-Wasser"), 5 µl 10 x AP-Puffer (Boehringer Mannheim), 1 µl 50 mM Dithiothreitol und 1,5 µl RNasin (Promega) gelöst. Nach Zugabe von 4 µl alkalischer Phosphatase (AP; 1U/µl; Boehringer Mannheim) wurde 1 h bei 37 °C inkubiert. Zur Inaktivierung der Phosphatase wurde mit µg Proteinase K (Boehringer Mannheim) versetzt und 1 h bei 37 °C inkubiert. Die Proben wurden wie oben beschrieben mit TE-gesättigtem Phenol und mit Chloroform/Isoamylalkohol extrahiert und mit Ethanol gefällt. Die Fällungen wurden in je 40 µl DEPC-Wasser gelöst.

Nach Zugabe von 5 µl 10 x TAP-Puffer, 1,25 µl RNasin, 1 µl 100 mM ATP und 3 µl Saurer Pyrophosphatase aus Tabak (TAP; 5 U/µl; Epicentre) wurde für 1 h bei 37 °C inkubiert, um ein "Decapping" zu bewirken. Die Reaktionsansätze wurden erst mit Phenol, dann mit Chloroform/Isoamylalkohol extrahiert und mit Ethanol gefällt. Die RNA wurde anschließend in 100 µl Elutionspuffer (Dynal) gelöst, und unter Einsatz von oligo(dT)-Dynabeads (Dynal) wurde die Poly(A)⁺ mRNA isoliert.

Nach Elution in 4,2 µl Wasser wurde mit 1,8 µl Ligationsmix (hergestellt aus 3 µl 10 x RNA-Ligase-Puffer [Boehringer Mannheim], 0,75 µl RNasin, 1,5 µl 2 mM ATP, 1,5 µl T4-RNA-Ligase [10 U/µl; Boehringer Mannheim] und 1 µg Anker-RNA-Oligonucleotid 5'-CAC GAU CUG AGG CCG AUC GAU UCA-3' in 1 µl H₂O) versetzt und 20 h bei 16 °C ligiert. Der Ligationsansatz wurde mit Ethanol gefällt und die Fällung in 2,8 µl DEPC-Wasser aufgenommen.

Zur reversen Transkription wurden 1,5 µl einer Mischung aus 4,3 µl 5 x RT-Puffer (Boehringer Mannheim), 1 µl einer 20 mM-Lösung von dATP, dTTP, dGTP und dCTP, 1 µl cDNA-Primer CT29V (30 pmol/µl; 5'-ACC TAC GTG CAG AT₁₅V-3' mit V=G, A oder C) und 1 µl M-MuL V-Reverse Transkriptase (20 /µl; Boehringer Mannheim) hinzugegeben, und es wurde 1 h bei 37 °C und nachfolgend 30 min. bei 42 °C inkubiert. Die Reaktionen wurden mit TE-Puffer auf 20 µl aufgefüllt.

Zur cDNA-Zweitstrangsynthese und Amplifikation wurden je 2 µl des so gewonnenen "Anker-cDNA-Pools" mit 28,5 µl H₂O, 5 µl 10 x PCR-Puffer (670 mM Tris/HCl, pH 8,8; 170 mM (NH₄)₂SO₄; 1 % Tween 20), 5 µl einer 1 mM-Lösung von dATP, dTTP, dGTP und dCTP, 8 µl 10 mM MgCl₂, je 0,1 pmol Primer CT29V und AP23 (5'-CAC GAT CTG AGG CCG ATC GAT TC-3') in 1 µl H₂O und 0,5 µl Taq-DNA-Polymerase (5 U/µl; Boehringer Mannheim) auf Eis vermischt. Die Reaktionen wurden mit Paraffinöl überschichtet und in einen auf 95 °C vorgeheizten Trioblock Thermocycler (Biometra) gesetzt. Nach anfänglicher Denaturierung für 5 min. bei 95 °C wurden 20 Zyklen des folgenden Amplifikationsprogramms durchgeführt: 30 sec. Denaturierung bei 95 °C, 2 min. Annealing und Extension bei 72 °C. Die Reaktionen wurden auf QiaQuick-Säulen (Quiagen) gereinigt; die Elution erfolgte mit 20 µl TE. Nach Zugabe von 2,3 µl 10 x Puffer A (Boehringer Mannheim) und 1 µl Sau3AI (4 U/µl; Boehringer Mannheim) wurde 1,5 h bei 37 °C gespalten. Die Reaktionen wurden mit Ethanol gefällt.

Jeweils 1,5 nmol der Oligonucleotide LT20 (5'-TCA CAT GCT AAG TCT CGC GA-3') und LT24 (5'GAT CTC GCG AGA CTT AGC ATG TGA C-3') wurden in einem Volumen von 50 µl H₂O miteinander vermischt und für 3 min. auf 90 °C erwärmt. Die Reaktionsmischung wurde auf 50 mM Tris/HCl (pH 7,5) und 10 mM MgCl₂ eingestellt und zur Generation doppelsträngiger Linker langsam auf 4 °C abgekühlt.

Die gefällten Restriktionsfragmente wurden unter Zugabe von 0,5 µl der wie beschrieben hergestellten Linker in 4 µl Gesamtvolumen einer Ligationsmischung nach Cobianchi und Wilson (Methods Enzymol. 152 (1987), 94-110) aufgenommen und 16 h bei 16 °C ligiert. Die unligierten Linker wurden auf QiaQuick-Säulen entfernt.

Zur nachfolgenden Amplifikation wurde jeweils ein Fünftel der gereinigten Ligationsreaktion mit 10 µl 10 x PCR-Puffer, 16 µl 10 mM MgCl₂, 5 µl 1 mM dATP, dTTP, dGTP und dCTP, je 20 pmol CT29V und LT24 in einem Gesamtvolumen von 100 µl vereint. Nach Erwärmung für 5 min auf 95 °C im Thermocycler wurden 5 U Taq-DNA-Polymerase hinzugegeben und unter folgenden Bedingungen 20 Zyklen einer Amplifikation durchgeführt: 30 sec. Denaturierung bei 95 °C, 90 sec. Annealing und Extension bei 72 °C.

Jeweils 1 µl Amplifikationsprodukt wurde für eine zweite Amplifikationsrunde eingesetzt. Diese erfolgte nach den obigen Bedingungen, aber in einem Gesamtvolumen von 30 µl für 30 Zyklen und unter Kombination jeweils eines der mit Digoxigenin markierten Primer DIG-CT29A (sequenzidentisch mit CT29V, aber statt degenerierter letzter Base "V" ein "A" als eindeutige letzte Base), DIG-CT29C oder DIG-CT29G mit jeweils einem der 16 möglichen, gegenüber LT24 um zwei Basen verlängerten Primer LT24NN. Jeweils 3 µl einer Reaktion wurden mit 1,5 µl Cycle Sequencing Stoplösung von Promega versetzt.

Dann wurden einander entsprechende Präparationen der zu vergleichenden Gewebe nebeneinander auf zwei Spuren eines denaturierenden 3,5 %-Polyacrylamidgels geladen. Auf eine freie Spur wurde als Längenstandard DIGmarkierter DNA Molecular Weight Marker VIII (Boehringer Mannheim) aufgetragen. Die elektrophoretische Auftrennung und die Übertragung auf eine Nylonmembran erfolgten mittels einer GATC 1500-Sequenzierapparatur (MWG Biotech) gemäß den Anweisungen des Herstellers. Die Nylonmembran wurde unfixiert gemäß dem Herstellerprotokoll zum Chemilumineszenz-Nachweis von Nucleinsäuren behandelt. Nach Exposition auf Röntgenfilm (Kodak X-Omat) wurden Membran und Film zur Deckung gebracht, und differenziell erscheinende Banden wurden mittels eines Skalpells aus der Membran ausgeschnitten. Das entsprechende Membranstückchen wurde in einer 50 µl-Reamplifikationsreaktion unter den obigen Bedingungen inkubiert; dabei wurden 30-40 Zyklen angewendet. Die erfolgreiche Reamplifikation sowie die Reinheit des Reamplifikationsprodukts wurde durch Agarosegelelektrophorese überprüft.

### Beispiel 3:

### Analyse geschlechtsabhängiger MADS-Box-Genexpression

Die in Beispiel 1 aus männlichen und weiblichen Infloreszenzen isolierte mRNA wurde gemäß dem RACE-Protokoll (Frohman et al, Supra) in Erststrang-cDNA überführt, wobei cDNA-Pools erhalten wurden. Mit den cDNA-Pools wurde eine PCR unter Verwendung des bei Frohman et al, Supra beschriebenen RACE-Adapter-Primers und eines Gemisches von Genfamilien-spezifischen MADS-Box-Primern durchgeführt. Alle MADS-Box-Primer waren gegen Genfamilien-spezifische Sequenzen gerichtet, die für Derivate eines hochkonservierten Aminosäure-Motivs codieren, und umfaßten alle aus Pflanzen bekannten Variationen. Um eine möglichst hohe Spezifität zu erreichen, wurde der Degenerationsgrad so gering wie möglich gehalten. Die in dieser Arbeit verwendeten Primer waren wie folgt:

P038 (gerichtet gegen die Aminosäuresequenz "IKRIEN") 5'-GAT CAA G(A/C)G (G/C)AT CGA GAA-3'; P041 (gegen "MKRIEN"), 5'-GAT GAA G(A/C)G (G/C)AT CGA GAA-3'; P042 (gegen "IKHIEN"), 5'-GAT CAA GCA (C/T)AT CGA GAA-3'; P043 (gegen "IKKIEN"), 5'-GAT CAA GAA GAT CGA GAA-3'.

Mit Primer P038 wurde die Amplifikation wie folgt durchgeführt:

Zunächst wurde ein 50 µl-Reaktionsgemisch auf Eis zusammenpipettiert, das 67 mM Tris/HCl, pH 8,8; 17 mM (NH₄)₂SO₄;0,1 % Tween 20; 0,8 mM MgCl₂; jedes dNTP mit 20 µM; 1,5 pmol des Primers P038 und 1 µl des jeweiligen cDNA-Pools enthielt. Nach Überschichten mit Paraffinöl wurden die Reaktionsmischungen in einem Trioblock Thermocycler (Biometra, Göttingen) für 5 min auf 95 °C erhitzt. Nach Abkühlen auf 65 °C wurden 2,5 U Taq DNA-Polymerase (Boehringer Mannheim) zugegeben. Es wurden 30 Zyklen der linearen Amplifizierung unter Verwendung der folgenden Parameter durchgeführt: Denaturierung für 30 sec. bei 95 °C, Annealing für 100 sec. bei 54 °C, Extension für 2 min. bei 72 °C. Anschließend wurden die Reaktionsgemische auf 65 °C abgekühlt.

Für eine exponentielle Amplifikation wurden 50 µl einer auf 65 °C vorgewärmten Reaktionsmischung, die 100 µM dNTPs, 15 pmol RACE Adapterprimer und Primer P038 und 2,5 U Taq DNA-Polymerase enthielt, den linearen Amplifikationsreaktionen zugesetzt. Dann wurde eine PCR unter Verwendung der oben genannten Parameter für 24 Zyklen durchgeführt, gefolgt von einer abschließenden Extension für 10 min bei 72 °C. Die PCR-Produkte wurden auf QiaQuick-Säulen (Quiagen, Hilden) gereinigt. 1/5 (10 µl) des Eluats wurde mit 15 U der Restriktionsenzyme Mse I (New England Biolabs), Hinfl oder Sau3AI (Boehringer Mannheim) in 50 µl des jeweiligen Restriktionspuffers für 3 hr bei 37 °C inkubiert. Nach einer anschließenden Inkubation für 10 min. bei 65 °C wurden die Nucleinsäuren mit Ethanol gefällt und durch Zentrifugation (30 min, 20.000 x g bei 4 °C) isoliert. Der Rückstand wurde in 5 µl TE resuspendiert.

Sofern gewünscht, können bereits in diesem Stadium radioaktive Primerextensionsreaktionen, wie weiter unten beschrieben, durchgeführt werden. Andererseits kann zur Reamplifikation eine Linkerligation, wie im folgenden beschrieben, erfolgen. Hierzu wurde 1/2 µl der resuspendierten Restriktionsfragmente in 10 µl Auffüllungsansätzen (50 mM Tris/HCl, pH 8,0; 6 mM MgCl₂; 10 mM Dithioerythritol; jedes dNTP mit 100 µM; 40 µg Rinderserumalbumin pro ml; 0,5 U T4-DNA-Polymerase (Boehringer Mannheim)) für 1 h bei 37 °C inkubiert. Die Fragmente wurden dann mit Ethanol gefällt und - wie oben beschrieben - isoliert. Die Pellets wurden in 2 µl TE-Puffer resuspendiert.

Aus jeweils 1,25 nmol der Oligonucleotide LR32 (5'-ACT CGA TTC TCA ACC CGA AAG TAT AGA TCC CA-3') und BTL18 (5'-TGG GAT CTA TAC TTT CAA-3') wurden wie in Beispiel 2 beschrieben doppelsträngige Linker hergestellt. Die Ligation der Linker an die endaufgefüllten Restriktionsfragmente erfolgte wie in Beispiel 2 beschrieben; dabei wurden jeweils 0,5 µl der Linker und 2 µl resuspendierte Endauffüllungsreaktion in 4 µl Ligationspuffer inkubiert. Die unligierten Linker wurden auf QuiaQuick-Säulen entfernt.

Dann erfolgte eine Reamplifikation unter den oben genannten PCR-Bedingungen unter Verwendung von 5 µl gereinigter Ligationsmischung als Matrize in insgesamt 15 Zyklen. Gemäß der dem "fmol DNA sequencing system" (Promega) beiliegenden Vorschrift wurde der jeweilige MADS-Box Primer (z. B., P038) mit ³²P markiert. Dann wurde ein halber Mikroliter der Reamplifikationsprodukte zur Herstellung einer 10 µl-Primer-Extensionsmischung verwendet, die 50 µM dNTPs, 1 U Taq-DNA-Polymerase und 0,4 µl markierten MADS-Box Primer enthielt. Die Reaktionsmischungen wurden in die Vertiefungen eines vorgeheizten (95 °C) Termocycler gegeben und anfänglich für 3 min bei 95 °C denaturiert, gefolgt von einem Programm von 30 sec. bei 95 °C, 30 sec. bei 54 °C und 1 min bei 72 °C über 30 Zyklen. Nach der abschließenden Extension wurden 5 µl der Cycle Sequencing Stoplösung von Promega zugegeben. Als Längenstandard wurde eine "Sequamark" 10-bp-Leiter (Research Genetics, Huntsville, AL) hergestellt. Zur gelelektrophoretischen Analyse wurde die Extensionsmischung für 2 min auf 70 °C erhitzt und 2 µl wurden auf ein denaturierendes 5 %-Acrylamidgel aufgegeben. Die Gele wurden für 3 h bei 45 W laufengelassen, in 5 % Essigsäure/10 % Ethanol fixiert und an der Luft getrocknet. Dann wurden die Gele für 4-12 h auf einen Kodak X-Omat Röntgenfilm belichtet.

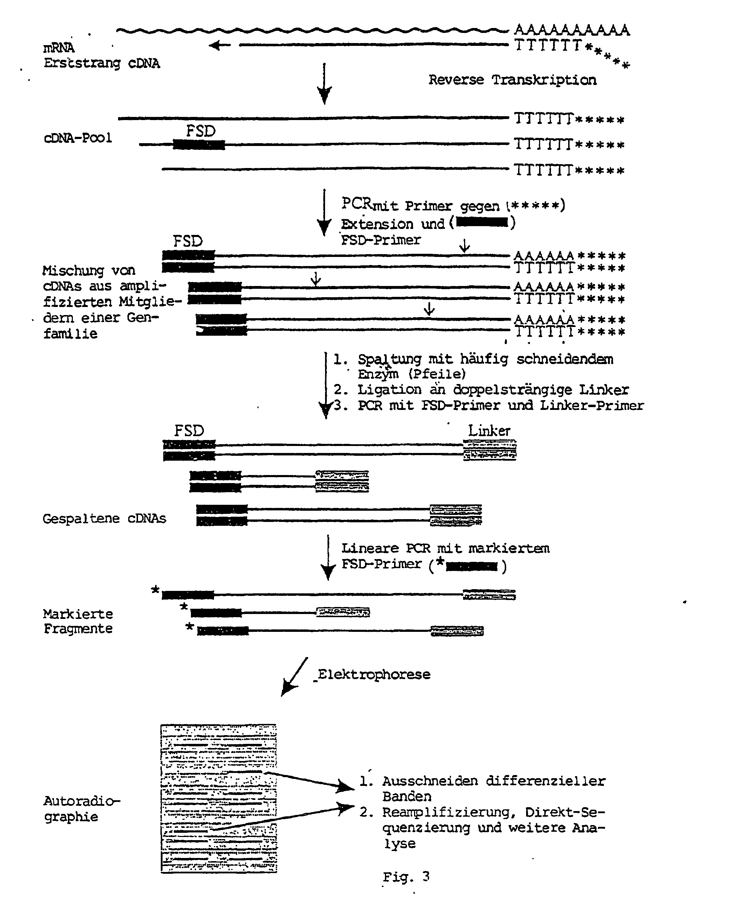

Eine schematische Darstellung des in Beispiel 3 durchgeführten Reaktionsprotokolls ist in Fig. 3 angegeben.

### Beispiel 4:

### Vergleich der MADS-Box-Genexpression zwischen Teosinte und Mais

Die in Beispiel 1 aus weiblichen Infloreszenzen von Mais und Teosinte isolierte DNA wurde wie in Beispiel 3 beschrieben in Erststrang-cDNA umgeschrieben und zur Synthese von Amplifikationsprodukten transkriptspezifischer Länge eingesetzt. Dabei kam zur selektiven Amplifikation von cDNA-Molekülen, die Mitglieder der MADS-Box-Genfamilie repräsentieren, eine Mischung der Oligonucleotide P038, P041, P042 und P043 zum Einsatz, die in diesem Fall an ihrem 5'-Ende eine Biotingruppe trugen. Nach der Inkubation mit Msel als Restriktionsenzym wurden die Biotinmarkierten Extensionsprodukte mittels Streptavidin-beschichteter Magnetpartikel (Dynal) abgetrennt und wie in Beispiel 2 und 3 beschrieben zur Ligation von Linkern eingesetzt. Zur Reamplifikation kam Oligonucleotid P046 5'-(A/C) G(G/C) CAG GT(G/C) AC(C/G) T(A/T)C-3' (gegen "RQVT(F/Y)" gerichtet) zum Einsatz). Die so erhaltenen Amplifikationsprodukte wurden wie beschrieben radioaktiv markiert und auf einem 6%igen Sequenziergel aufgetrennt.

### Beispiel 5:

### Analyse und Identifizierung der Amplifikationsprodukte

Die interessierenden Banden wurden aus den in Beispiel 3 und Beispiel 4 erhaltenen Gelen ausgeschnitten. Die darin enthaltenen Nucleinsäuren wurden in 25 µl TE-Puffer eluiert. Jeweils 5 µl Eluat wurden in einem Volumen von 50 µl unter Verwendung der oben genannten PCR-Bedingungen in 30 Zyklen reamplifiziert. Als Primer wurden der jeweilige MADS-Box Primer und das Oligonucleotid LR21, das mit den Basen 1-21 von LR32 identisch ist, verwendet. Zur Sequenzanalyse wurden 0,5 µl der reamplifizierten Bande gemäß den Vorschriften des pmol Cycle-Sequencing-Kits von Promega sequenziert, wobei als Primer entweder LR32, der jeweils verwendete MADS-Box PCR-Primer oder der interne MADS-Box Primer P009 (5'-GAA GGC GTA CGA GCT CTC GGT GCT-3') verwendet wurde. Die Annealing-Temperaturen waren 70 °C für LR32 und P009 bzw. 50 °C für die anderen MADS-Box Primer. Die Sequenzinformation aus den ausgeschnittenen Banden wurde dann zur Identifizierung entsprechender Klone in cDNAoder Genbanken verwendet.

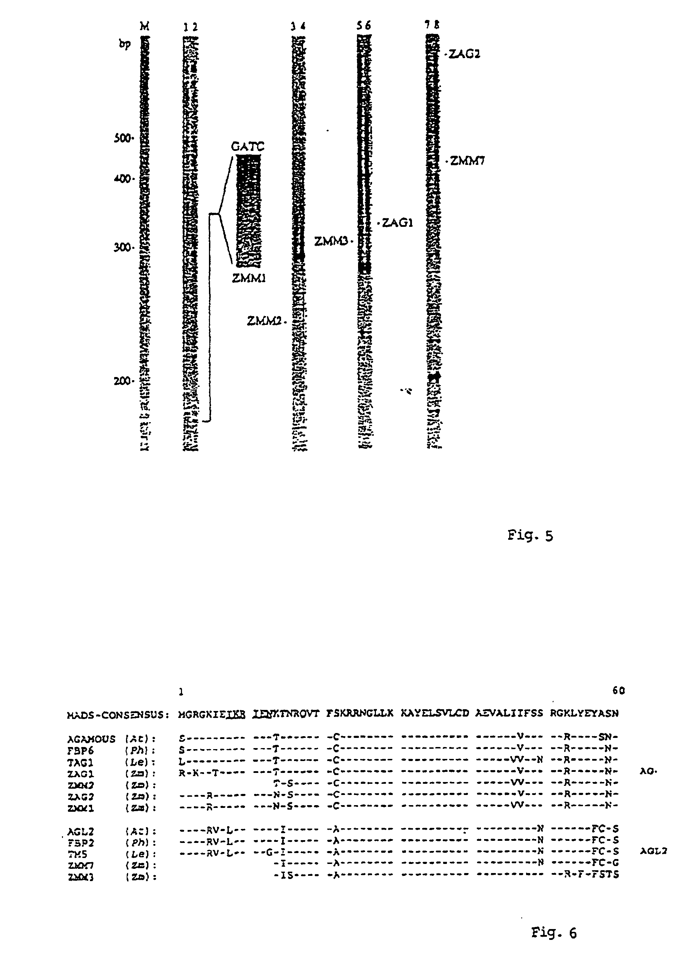

In Fig. 5 ist das-Ergebnis der Isolierung geschlechtsabhängig exprimierter MADS-Box-Gene gezeigt. Die Spuren mit ungeraden Zahlen entsprechen Transkripten von männlichen Infloreszenzen, die Spuren mit geraden Zahlen entsprechen weiblichen Transkripten. Das Gen ZMM1 wurde unter Verwendung des Primers P038 und Restriktionsenzyms Sau3AI erhalten, die Sequenzleiter zeigt einen Teil der ZMM1-Sequenz, die durch direkte Sequenzierung der ausgeschnittenen und reamplifizierten Bande mit LR32 als Sequenzierungsprimer erhalten wurde. Zur Identifizierung von ZMM2 wurden der Primer P042 und das Enzym Hinfl verwendet. ZMM3 und ZAG1 wurden unter Verwendung des Primers P041 und Msel erhalten. ZMM7 und ZAG2 wurden mit dem Primer P043 und Msel erhalten. Die mit M bezeichnete Spur stellt den Nucleinsäurenlängenmarker dar

In Fig. 6 ist eine Zuordnung der durch das erfindungsgemäße Verfahren identifizierten Gene gezeigt. Ein Vergleich von 101 bekannten Sequenzen von MADS-Domänen aus Hefe, Tieren und Pflanzen ergab die in Zeile 1 angegebene MADS-Domänen-Konsensus-Sequenz. Weiterhin wurden unterschiedliche Subfamilien gefunden, die sich hinsichtlich der gesamten Gensequenz, des Expressionsmusters und der Funktion unterscheiden. Die durch das erfindungsgemäße Verfahren identifizierten MADS-Sequenzen aus Mais sind Mitglieder von zwei dieser Subfamilien, der AGartigen und der AGL2-artigen Subfamilie. Die Vergleichssequenzen AGAMOUS (Yanofsky et al, Nature 346 (1990), 35-39) und AGL2 (Ma et al, Genes Dev. 5 (1991), 484-495) stammen von Arabidopsis thaliana (At). Die Vergleichssequenzen FBP6 (Angenent et al, Plant J. 4 (1993), 101-112) und FBP2 (Angenent et al, Plant Cell 4 (1992), 983-993) stammen von Petunia hybrida (Ph). Weiterhin sind als Vergleichssequenzen TAG1 (Pnueli et al, Plant Cell 6 (1994), 163-167) und TM5 (Pnueli et al, Plant J. 1 (1991), 255-266) aus Lycopersicon esculentum (Le) angegeben. Im vorliegenden Versuch wurden die Gene ZAG1, ZAG2, ZMM1 und ZMM2 (Schmidt et al), Plant Cell 5 (1993), 729-737; Theissen et al, Gene 156 (1995), 155-166) sowie weitere neue Sequenzen ZMM3 und ZMM7 aus Zea mays (Zm) identifiziert. Für die einzelnen Sequenzen der MADS-Domäne in Fig. 6 sind nur die Abweichungen von der Konsensus-Sequenz angegeben, wobei die Bindestriche eine Identität mit der Konsensus-Sequenz anzeigen. Die unterstrichenen Aminosäuren in der Konsensus-Sequenz zeigen die Bindungsstelle für den Genfamilien-spezifischen Primer.

Bei allen sechs identifizierten MADS-Box-Genen aus Mais konnte die durch das erfindungsgemäße Verfahren nachgewiesene differenzielle Expression durch unabhängige Northern Blot oder in situ Hybridisierungsexperimente bestätigt werden. Ein Vergleich der Bandenintensitäten der Amplifikationsprodukte mit entsprechenden Daten aus Northern Blots zeigt, daß das erfindungsgemäße Verfahren eine zumindest semiquantitative Bestimmung der Expression einzelner Mitglieder von Genfamilien in unterschiedlichen Proben ermöglicht.

Weiterhin besitzt das erfindungsgemäße Verfahren eine hohe Reproduzierbarkeit, da 10 unabhängige Wiederholungsexperimente ausgehend von der cDNA exakt die gleichen Bandenmuster ergaben.

Insgesamt konnten durch das erfindungsgemäße Verfahren sechs unterschiedliche MADS-Box-Gene identifiziert werden, von denen 4 bereits bekannt waren. Von den bereits bekannten Genen konnte die Expression von ZAG2 und ZMM1 als spezifisch für weibliche Pflanzen identifiziert werden. ZAG1 wird stärker in weiblichen Pflanzen als in männlichen Pflanzen exprimiert. ZMM2 wird hingegen stärker in männlichen als in weiblichen Infloreszenzen exprimiert.

Die zwei neuen, durch das erfindungsgemäße Verfahren isolierten MADS-Box-Gene gehören zur Subfamilie der AGL2-artigen Gene. Eines dieser Gene, bezeichnet als ZMM3, wird stärker in männlichen als in weiblichen Infloreszenzen exprimiert. Die Expression des neuen Gens ZMM7 scheint hingegen spezifisch in weiblichen Pflanzen stattzufinden.
## Patentansprüche

1. Verfahren zur Genexpressionsanalyse, umfassend folgende Schritte:
(a) Isolieren von mRNA-Molekülen aus einer oder mehreren zu analysierenden Gewebeproben,
(b) Synthetisieren von cDNA-Erststrangmolekülen aus den mRNA-Molekülen,
(c) Synthetisieren von cDNA-Zweitstrangmolekülen,
(d) Spalten der cDNA-Moleküle aus (c) mit mindestens einer Restriktionsendonuklease,
wobei die Restriktionsendonuklease eine vierbasige Erkennungssequenz aufweist,
(e) Anfügen von Linkem an die durch den Restriktionsverdau gewonnene cDNA-Fragmente,
(f) Selektive Amplifikation eines Teils der Ligationsprodukte aus (e),
wobei die Amplifikation mittels der Polymerasekettenreaktion erfolgt und mindestens ein gegen die angefügten Linker gerichteter Primer sowie mindestens ein gegen die Sequenz des in Schritt (b) eingesetzten cDNA-Primers gerichteter Primer eingesetzt wird, und wobei die eingesetzten Primer gegebenenfalls an ihrem 3'-Ende um eine Extension von einer oder mehreren selektiven Basen verlängert sind,
(g) Auftrennen der Amplifikationsprodukte nach ihrer Länge, wobei die Amplifikationsprodukte an ihren Enden Sequenzen enthalten, an die die in Schritt (f) verwendeten Primer binden können, und
(h) Analysieren der Amplifikationsprodukte.

2. Verfahren nach Anspruch 1,
**dadurch gekennzeichnet,**
**dass** in Schritt (c) eine Amplifikation der cDNA-Moleküle erfolgt.

3. Verfahren nach einem der Ansprüche 1 oder 2, weiterhin umfassend einen Schritt:
(i) Vergleichen der aus verschiedenen mRNA-Präparationen gewonnenen Mischungen von Amplifikationsprodukten.

4. Verfahren nach einem der Ansprüche 1 bis 3,
**dadurch gekennzeichnet,**
**dass** der in Schritt (b) zur Synthese eingesetzte oligo(dT)-Primer an seinem 3'-Ende und/oder an seinem 5'-Ende eine Extension aufweist.

5. Verfahren nach einem der Ansprüche 1 bis 4,
**dadurch gekennzeichnet,**
**dass** die Auftrennung der Amplifikationsprodukte in Schritt (g) durch Direkt-Blotting-Elektrophorese erfolgt.

6. Verfahren nach einem der Ansprüche 1 bis 5,
**dadurch gekennzeichnet,**
**dass** die Amplifikationsprodukte aus Schritt (f) fluoreszente Markierungsgruppen tragen und Auftrennung und Nachweis mittels einer automatisierten Sequenzapparatur erfolgen.

7. Verfahren nach einem der Ansprüche 1 bis 6,
**dadurch gekennzeichnet,**
**dass** die eingesetzten Oligonukleotide eine oder mehrere Erkennungsstellen für Restriktionsenzyme enthalten.

8. Verwendung von gemäß Anspruch 1, Schritte (a) bis (g) hergestellten cDNA-Restriktionsfragmenten zur vergleichenden Expressionsanalyse zweier oder mehrerer Proben, wobei die aus unterschiedlichen Proben gewonnenen Fragmentmischungen miteinander verglichen und Fragmente identifiziert werden, welche zwischen den zu vergleichenden Proben als differenziell erscheinend detektiert werden.

9. Reagenzienkit zur Analyse der Expression von Genen in einem Verfahren nach einem der Ansprüche 1 bis 7, umfassend:
(a) ein Enzym zur cDNA-Erststrangsynthese,
(b) mindestens ein Enzym zur Synthese von cDNA-Zweitsträngen und zur Amplifikation von DNA-Fragmenten,
(c) einzelsträngige Nucleinsäure-Primermoleküle ausgewählt aus der Gruppe, umfassend (i) mindestens ein Oligo-dT-Nucleotid, (ii) Primer gemäß (i) mit einer 3'-Extension oder/und 5'-Extension, wobei einer der Primer gemäß (i-ii) eine Markierungsgruppe ausgewählt aus Fluoreszenzfarbstoffen, Biotin oder Antigenen trägt, und (iii) Primer gemäß (i) oder (ii), die eine durch die Art ihrer Markierung, z.B. die Farbe einer Fluoreszenzmarkierung, codierte 3'-Extension enthalten, und
(d) Mittel zur Markierung und zum Nachweis von Nucleinsäuren.

10. Reagenzienkit nach Anspruch 9, weiterhin umfassend:
(e) mindestens ein Restriktionsenzym,
(f) mindestens ein doppelsträngiges DNA-Linkermolekül und Mittel zur Befestigung des Linkermoleküls an den Enden von DNA-Fragmenten,
(g) mindestens ein einzelsträngiges RNA-Oligonucleotid und Mittel zur Befestigung des RNA-Oligonucleotids am 5'-Ende von mRNA-Molekülen, oder/und
(h) weitere Reagenzien wie RNase-Inhibitor, Puffer, Streptavidin-beschichtete Reaktionsgefäße oder magnetische Partikel.

11. Anwendung des Verfahrens nach einem der Ansprüche 1 bis 7 zur Isolierung neuer Gene.

12. Anwendung des Verfahrens nach einem der Ansprüche 1 bis 7 zur Identifizierung neuer Mitglieder von Genfamilien.

13. Anwendung des Verfahrens nach einem der Ansprüche 1 bis 7 zur Bestimmung des Lebensstadiums einer Zelle oder eines Organismus.

14. Anwendung des Verfahrens nach einem der Ansprüche 1 bis 7 zur Untersuchung des Lebenszyklus einer Zelle oder eines Organismus.

15. Anwendung nach einem der Ansprüche 11 bis 14 für die Krebs- oder Altersforschung.

## Claims

1. Method for gene expression analysis comprising the following steps:
(a) isolation of mRNA molecules from one or more tissue samples to be analysed,
(b) synthesising cDNA first strand molecules from the mRNA molecules,
(c) synthesising cDNA second strand molecules,
(d) cleaving the cDNA molecules from (c) with at least one restriction endonuclease, the restriction endonuclease exhibiting a four-base recognition sequence;
(e) attaching linkers to the cDNA fragments obtained by restriction digestion,
(f) selective amplification of a part of the ligation products from (e) wherein the amplification is carried out by means of the polymerase chain reaction and at least one primer directed against the attached linkers and at least one primer directed against the sequence of the cDNA primer used in step (b), and wherein the primers used are optionally extended at their 3' end by an extension of one or more selective bases,
(g) separation of the amplification products according to their length, the amplification products having sequences at their ends to which the primers used in step (f) can bind, and
(h) analysis of the amplification products.

2. Method according to claim 1, **characterised in that** the cDNA molecules are amplified in step (c).

3. Method according to either claim 1 or claim 2, further comprising a step:
(i) comparing mixtures of amplification products obtained from various mRNA preparations.

4. Method according to any one of claims 1 to 3, **characterised in that** the oligo (dT) primer used for the synthesis in step (b) has an extension at its 3' end and/or at its 5' end.

5. Method according to any one of claims 1 to 4, **characterised in that** the amplification products are separated in step (g) by direct blotting electrophoresis.

6. Method according to any one of claims 1 to 5, **characterised in that** the amplification products from step (f) carry fluorescent marker groups and the separation and detection are carried out by means of an automated sequencing apparatus.

7. Method according to any one of claims 1 to 6, **characterised in that** the oligonucleotides that are used contain one or more recognition sites for restriction enzymes.

8. Use of cDNA restriction fragments produced according to claim 1, steps (a) to (g), for the comparative expression analysis of two or more samples, wherein the fragment mixtures obtained from different samples are compared with one another and fragments are identified which are detected by their differential appearance when the samples are compared.

9. Reagent kit for the analysis of the expression of genes in a method according to any one of claims 1 to 7, comprising:
(a) an enzyme for cDNA first strand synthesis,
(b) at least one enzyme for the synthesis of cDNA second strands and for the amplification of DNA fragments,
(c) single-stranded nucleic acid primer molecules selected from the group comprising (i) at least one oligo-dT nucleotide, (ii) primers according to (i) with a 3' extension or/and 5' extension, wherein one of the primers according to (i-ii) carries a marker group selected from fluorescence dyes, biotin or antigens, and (iii) primers according to (i) or (ii), which contain a 3' extension coded by the type of label, for example the colour of a fluorescence label, and
(d) agent for labelling and for detecting nucleic acids.

10. Reagent kit according to claim 9, addidonally comprising:
(e) at least one restriction enzyme,
(f) at least one double-stranded DNA linker molecule and an agent for attaching the linker molecule to the ends of DNA fragments,
(g) at least one single-stranded RNA oligonucleotide and an agent for attaching the RNA oligonucleotide to the 5' end of mRNA molecules or/and
(h) additional reagents such as RNase inhibitors, buffers, streptavidin-coated reaction vessels or magnetic particles.

11. Use of the method according to any one of claims 1 to 7 to isolate new genes.

12. Use of the method according to any one of claims 1 to 7 to identify new members of gene families.

13. Use of the method according to any one of claims 1 to 7 to determine the living stage of a cell or an organism.

14. Use of the method according to any one of claims 1 to 7 to examine the living cycle of a cell or an organism.

15. Use according to any one of claims 11 to 14 for cancer or geriatric research.

## Revendications

1. Procédé d'analyse de l'expression génétique comportant les étapes suivantes :
(a) Isolation de molécules d'ARNm à partir d'un ou plusieurs échantillons de tissu à analyser,
(b) Synthèse de molécules du premier brin d'ADNc à partir des molécules d'ARNm,
(c) Synthèse de molécules du deuxième brin d'ADNc,
(d) Clivage de la molécule d'ADNc de (c) avec au moins une endonucléase de restriction,
l'endonucléase de restriction présentant une séquence de reconnaissance à 4 bases,
(e) Ajout de linkers aux fragments d'ADNc obtenus par la digestion de restriction,
(f) Amplification sélective d'une partie des produits de ligature de (e),
l'amplification étant effectuée au moyen de la réaction en chaîne par polymérase et au moins une amorce dirigée contre les linkers ajoutés ainsi qu'au moins une amorce dirigée contre la séquence de l'amorce d'ADNc mise en oeuvre à l'étape (b) étant utilisées, et les amorces mises en oeuvre étant prolongées, le cas échéant, à leur extrémité 3', d'une extension d'une ou plusieurs bases sélectives,
(g) Séparation des produits de l'amplification selon leur longueur, les produits de l'amplification contenant, sur leurs extrémités, des séquences sur lesquelles les amorces utilisées à l'étape (f) peuvent se lier et
(h) Analyse des produits d'amplification.

2. Procédé selon la revendication 1,
**caractérisé en ce qu'**
à l'étape (c), une amplification des molécules d'ADNc est effectuée.

3. Procédé selon l'une quelconque des revendications 1 ou 2, comportant par ailleurs, une étape :
(i) Comparaison des mélanges de produits d'amplification obtenus à partir de différentes préparations d'ARNm.

4. Procédé selon l'une quelconque des revendications 1 à 3,
**caractérisé en ce que**
l'amorce oligo(dT) mise en oeuvre à l'étape (b) pour la synthèse présente une extension à son extrémité 3' et/ou à son extrémité 5'.

5. Procédé selon l'une quelconque des revendications 1 à 4,
**caractérisé en ce que**
la séparation de produits d'amplification à l'étape (g) est effectuée par électrophorèse de transfert direct.

6. Procédé selon l'une quelconque des revendications 1 à 5,
**caractérisé en ce que**
les produits d'amplification de l'étape (f) portent des groupes de marquage fluorescents et **en ce que** la séparation et la détection sont réalisées au moyen d'un appareillage de séquençage automatisé.

7. Procédé selon l'une quelconque des revendications 1 à 6,
**caractérisé en ce que**
les oligonucléotides mis en oeuvre contiennent un ou plusieurs points de reconnaissance pour des enzymes de restriction.

8. Utilisation de fragments de restriction d'ADNc obtenus selon la revendication 1, étapes (a) à (g), pour l'analyse comparative de l'expression de deux ou plusieurs échantillons, les mélanges de fragments obtenus à partir de différents échantillons étant comparés les uns aux autres et des fragments qui sont détectés comme apparaissant différents entre les échantillons à comparer, étant identifiés.

9. Kit de réactifs pour l'analyse de l'expression de gènes dans un procédé selon l'une quelconque des revendications 1 à 7 comportant :
(a) une enzyme pour la synthèse du premier brin d'ADNc,
(b) au moins une enzyme pour la synthèse de deuxièmes brins d'ADNc et pour l'amplification de fragments d'ADN,
(c) une molécule d'amorce d'acide nucléique simple-brin choisie dans le groupe comprenant (i) au moins un nucléotide oligo-dT, (ii) des amorces selon (i) ayant une extension 3' et/ou une extension 5', l'une des amorces selon (i-ii) portant un groupe de marquage choisi parmi les colorants fluorescents, la biotine ou des antigènes et (iii) des amorces selon (i) ou (ii) qui contiennent une extension 3' codée par le type de leur marquage, par exemple la couleur d'un marquage fluorescent et
(d) un moyen destiné au marquage et à la détection d'acides nucléiques.

10. Kit de réactifs selon la revendication 9, comprenant en outre :
(e) au moins une enzyme de restriction,
(f) au moins une molécule linker d'ADN double-brin et un moyen destiné à fixer la molécule linker aux extrémités de fragments d'ADN,
(g) au moins un oligonucléotide ARN simple-brin et un moyen destiné à fixer l'oligonucléotide ARN sur l'extrémité 5' de molécules d'ARNm et/ou
(h) d'autres réactifs comme un inhibiteur de la RNase, un tampon, des réacteurs recouverts de streptavidine ou des particules magnétiques.

11. Utilisation du procédé selon l'une quelconque des revendications 1 à 7 pour isoler de nouveaux gènes.

12. Utilisation du procédé selon l'une quelconque des revendications 1 à 7 pour identifier de nouveaux membres de familles de gènes.

13. Utilisation du procédé selon l'une quelconque des revendications 1 à 7 pour déterminer le stade de vie d'une cellule ou d'un organisme.

14. Utilisation du procédé selon l'une quelconque des revendications 1 à 7 pour étudier le cycle de vie d'une cellule ou d'un organisme.

15. Utilisation selon l'une quelconque des revendications 11 à 14 pour la recherche sur le cancer et le vieillissement.
